# EUROPEAN PATENT APPLICATION

(11) **EP 4 775 133 A1**
(43) Date of publication of application: **15.07.2026**
(21) Application number: 24891662.9
(22) Date of filing: 17.10.2024
(51) Int. Cl.: A61B 5/00, G16H 50/20, G16H 10/60

(54) **ELECTRONIC DEVICE AND DATA MANAGEMENT METHOD OF ELECTRONIC DEVICE**

(30) Priority: 17.11.2023 KR 20230159738; 27.12.2023 KR 20230193627
(71) Applicant: Samsung Electronics Co., Ltd., Suwon-si, Gyeonggi-do 16677 (KR)
(72) Inventor: CHO, Sunghwan, Suwon-si Gyeonggi-do 16677 (KR); PARK, Jeongmin, Suwon-si Gyeonggi-do 16677 (KR); KIM, Jaepil, Suwon-si Gyeonggi-do 16677 (KR); PARK, Byeongju, Suwon-si Gyeonggi-do 16677 (KR); YEO, Jaeyung, Suwon-si Gyeonggi-do 16677 (KR); LEE, Myeongjin, Suwon-si Gyeonggi-do 16677 (KR)
(74) Representative: Gulde & Partner
(86) International application number: PCT/KR2024/015817
(87) International publication number: WO 2025/105715

(57) **Abstract**

An electronic device according to an embodiment disclosed in the disclosure includes communication circuitry, a display, at least one sensor, a memory including a generative artificial intelligence model, and a processor. The memory stores instructions that, when executed by the processor, cause the electronic device to identify biometric data periodically measured using the at least one sensor, determine whether the biometric data is missing, identify information related to a period during which the biometric data was missing, identify a measurement history of previously measured biometric data, and, using the generative artificial intelligence model, generate missing biometric data based at least in part on the measurement history. In addition, various embodiments identified by the specification are also possible.

## Description

### [Technical Field]

Embodiments disclosed in the disclosure relates to a technology for detecting whether data obtained using a sensor is missing and managing the data.

### [Background Art]

Recent portable electronic devices (e.g., a mobile terminal or a wearable device) may provide various functions in a complex manner as well as a communication function with an external electronic device. For example, with the spread of various portable electronic devices, such as a smartphone and a wearable device, methods for measuring biometric information using the portable electronic device have been studied. The portable electronic device may include a sensor for measuring biometric information. The plurality of portable electronic devices (e.g., a mobile terminal and a wearable device) may share the measured biometric information with each other. As biometric information is measured using the portable electronic device, a health care service for a user may be provided. For example, the portable electronic device may periodically or aperiodically measure biometric information of the user, may identify a health state of the user, and may provide the user with information associated with the health state.

The information described above may be provided as the related art for the purpose of aiding the understanding of the disclosure. No assertion or determination is made with respect to the applicability of any of the above-mentioned contents as the prior art related to the disclosure.

### [Disclosure]

### [Technical Solution]

An electronic device according to an embodiment disclosed in the disclosure may include communication circuitry, a display, at least one sensor, a memory including a generative artificial intelligence mode, and a processor. The memory may store instructions that, when executed by the processor, cause the electronic device to identify biometric data periodically measured using the at least one sensor, determine whether the biometric data is missing, identify information related to a period during which the biometric data was missing, identify a measurement history of previously measured biometric data, and, using the generative artificial intelligence model, generate missing biometric data based at least in part on the measurement history.

Furthermore, a method for managing data of an electronic device according to an embodiment disclosed in the disclosure may include identifying biometric data periodically measured using at least one sensor, determining whether the biometric data is missing, identifying information related to a period during which the biometric data was missing, identifying a measurement history of previously measured biometric data, and generating, using a generative artificial intelligence model, missing biometric data based at least in part on the measurement history.

Furthermore, a storage medium according to an embodiment disclosed in the disclosure may store instructions or a program that, when executed by a processor of an electronic device, cause(s) the electronic device to identify biometric data periodically measured using at least one sensor, determine whether the biometric data is missing, identify information related to a period during which the biometric data was missing, identify a measurement history of previously measured biometric data, and, using the generative artificial intelligence model, generate missing biometric data based at least in part on the measurement history.

### [Description of Drawings]

FIG. 1 is a block diagram of an electronic device according to an embodiment;
FIG. 2 is a block diagram of a system according to an embodiment;
FIG. 3 is a block diagram of an electronic device according to an embodiment;
FIG. 4 is a drawing for describing an operation of generating missing biometric data in an electronic device according to an embodiment;
FIG. 5 is a drawing for describing an operation of detecting that data is missing in an electronic device according to an embodiment;
FIGS. 6A and 6B are drawings for describing an operation of generating and providing missing data in an electronic device according to an embodiment;
FIG. 7 is a drawing for describing an operation of generating and providing missing data in an electronic device according to an embodiment;
FIG. 8 illustrates an example of a user interface provided to manage data by an electronic device according to an embodiment;
FIG. 9 is a drawing for describing an operation of generating missing sensor data in an electronic device according to an embodiment;
FIG. 10 is a flowchart of a method for managing data of an electronic device according to an embodiment;
FIG. 11 is a flowchart of a method for managing data of an electronic device according to an embodiment;
FIG. 12 is a flowchart of a method for managing data of an electronic device according to an embodiment;
FIG. 13 is a flowchart of a method for managing data of an electronic device according to an embodiment;
FIG. 14 is a flowchart of a method for managing data of an electronic device according to an embodiment; and
FIG. 15 is a block diagram illustrating an electronic device in a network environment according to various embodiments.

With regard to description of drawings, the same or similar components will be marked by the same or similar reference signs.

### [Mode for Invention]

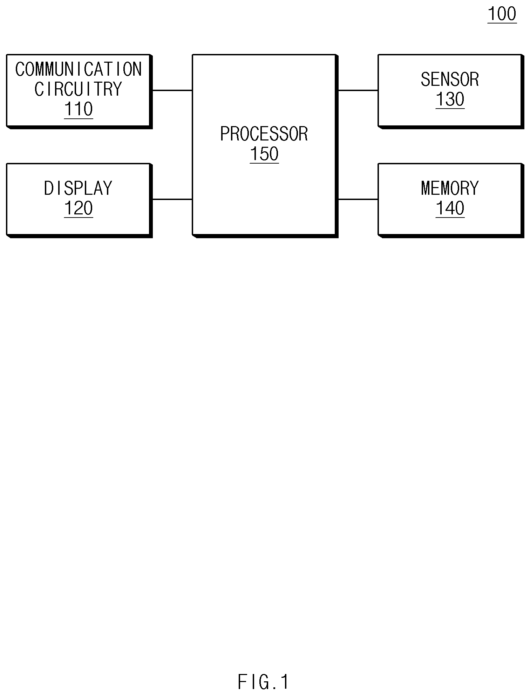

FIG. 1 is a block diagram of an electronic device according to an embodiment.

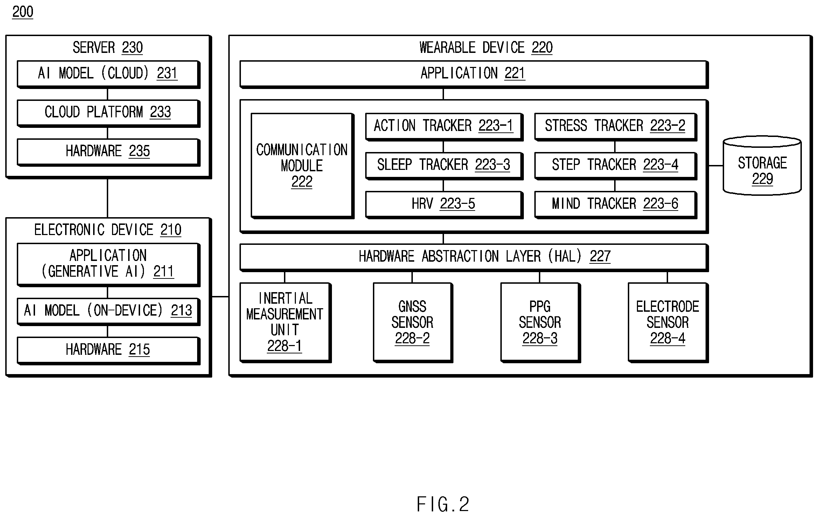

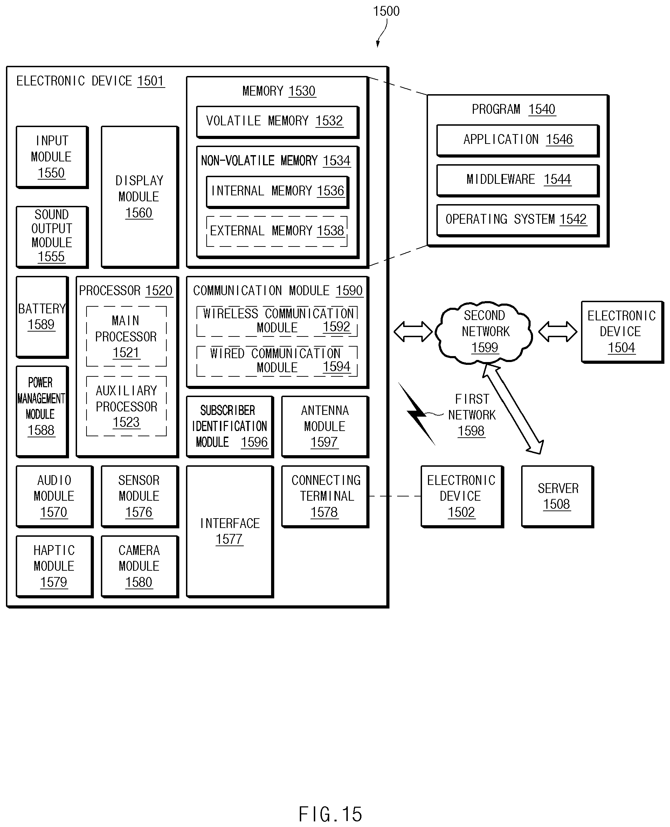

An electronic device 100 (e.g., an electronic device 210 of FIG. 2 or an electronic device 1501 of FIG. 15) according to an embodiment may include communication circuitry 110 (e.g., a communication module 1590 of FIG. 15), a display 120 (e.g., a display module 1560 of FIG. 15), at least one sensor 130 (e.g., sensors 228-1, 228-2, 228-3, and 228-4 of FIG. 2 or a sensor module 1576 of FIG. 15), a memory 140 (e.g., a memory 1530 of FIG. 15), and a processor 150 (e.g., a processor 1520 of FIG. 15).

According to an embodiment, the communication circuitry 110 may transmit and receive information and/or data with an external electronic device (not shown) (e.g., an electronic device 1502 or 1504 of FIG. 15) and/or an external server (not shown) (e.g., a server 1508 of FIG. 15). For example, the external electronic device may include a component which is substantially the same as the at least one electronic device 100 and may include a component different from the electronic device 100. For example, the communication circuitry 110 may receive biometric data of a user and/or sensor 130 data, which are/is obtained by the external electronic device 100, from the external electronic device 100. For example, the communication circuitry 110 may transmit the biometric data and/or the sensor 130 data to the external server and may receive missing biometric data and/or missing sensor 130 data, which are/is generated by the external server (e.g., a generative artificial intelligence model of the external server), from the external server.

According to an embodiment, the display 120 may visually output information. For example, the display 120 may output the biometric data and/or the sensor 130 data. For example, the display 120 may output an execution screen of an application and/or a user interface, which include(s) the biometric data and/or the sensor 130 data. For example, the display 120 may output a user interface including the generated missing data. According to an embodiment, the display 120 may output a user interface for confirming whether to store the generated missing data.

According to an embodiment, the at least one sensor 130 may measure biometric information of the user. For example, the at least one sensor 130 may be included in the outside of the electronic device 100 (e.g., the external electronic device 100).

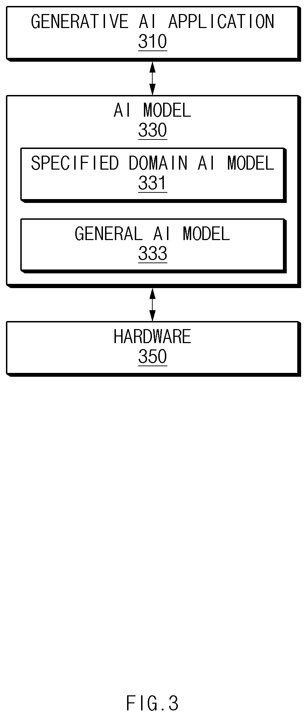

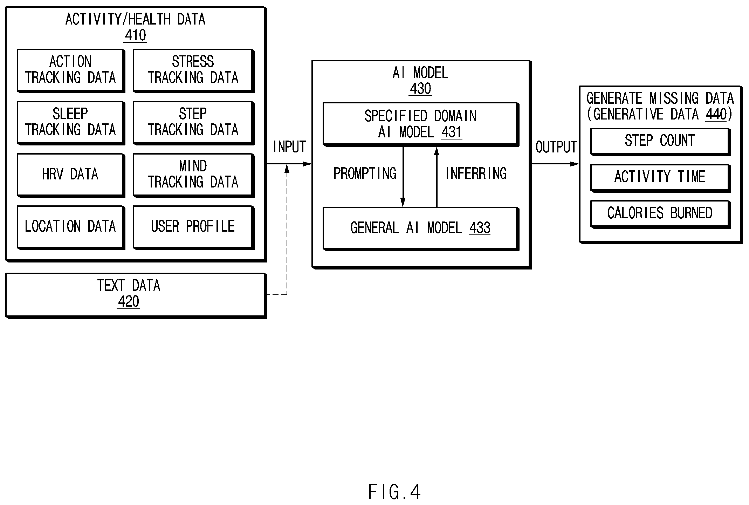

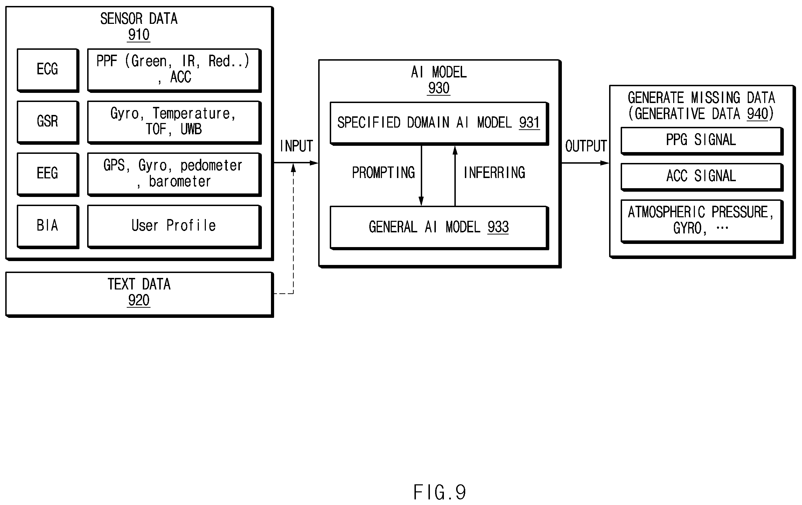

According to an embodiment, the memory 140 may store instructions that, when executed by the processor 150, control an operation of the electronic device 100. The memory 140 may include a generative artificial intelligence model (e.g., an AI model 213 of FIG. 2, an AI model 330 of FIG. 3, an AI model 430 of FIG. 4, or an AI model 930 of FIG. 9). The memory 140 may store sensor 130 data and/or biometric data of the user, which are/is measured using the sensor 130 of the electronic device 100 and/or the external electronic device 100. The memory 140 may store a user profile (e.g., a health state, body information, a name, a gender, an age, a preference of the user, activity history, and/or device usage history). The memory 140 may at least temporarily store text data obtained from a user input.

According to an embodiment, the processor 150 may identify biometric data periodically or aperiodically measured using the at least one sensor 130. For example, the electronic device may periodically or aperiodically measure biometric data. The electronic device may store biometric data measured using the sensor and may identify the stored biometric data. For example, a measurement period of the biometric data may include, but is not limited to, one day, one month, or one year and may be specified as any period. According to an embodiment, the processor 150 may receive biometric data measured using the sensor 130 of the external electronic device 100 (e.g., a wearable device) from the external electronic device 100.

According to an embodiment, the processor 150 may detect whether the biometric data is missing. For example, when the period is one day, the processor 150 may identify a day when the biometric data is missing (hereinafter, a "missing day"). According to an embodiment, the processor 150 may determine whether the biometric data is missing based on the result of comparing, for each specified cycle, a time when the biometric data is measured, an operation state of the electronic device 100 (e.g., a time when the electronic device 100 is powered on or a time when the electronic device 100 is powered off, or a time when the sensor 130 is activated/deactivated), or a time when the external electronic device 100 (e.g., the wearable device) is worn with a specified threshold (e.g., threshold time).

According to an embodiment, the processor 150 may identify information related to a period during which the biometric data was missing. For example, the processor 150 may identify information (e.g., a date, weather, a day of the week, and/or a year) related to a missing day of the biometric data.

According to an embodiment, the processor 150 may identify a measurement history of previously measured biometric data. For example, the processor 150 may identify a measurement history of biometric data measured before the missing day and/or a measurement history of biometric data measured after the missing day. For example, the processor 150 may identify a measurement history of biometric data measured during another period corresponding to the period.

According to an embodiment, the processor 150 may determine whether additional data is required to generate the missing biometric data. For example, when the additional data is required to generate the missing biometric data, the processor 150 may receive text data associated with behavior of the user during the period from the user. According to an embodiment, the processor 150 may obtain text data from the user through an input through a user interface, an input through a physical key, and/or a voice input through a microphone.

According to an embodiment, the processor 150 may generate the missing biometric data based at least in part on the measurement history of the biometric data and/or the text data, using the generative artificial intelligence model.

According to an embodiment, the processor 150 may obtain sensor 130 data measured using the plurality of sensors 130 during the specified period (which may be referred to as a "session" in the disclosure). For example, the specified period may be specified as a different period based on a user input, a state (e.g., log) of the electronic device 100, an application execution state, a state (e.g., an exercise state, a sleep state, or an activity state) of the user, which is determined by the electronic device 100.

According to an embodiment, the processor 150 may detect whether the sensor 130 data corresponding to the at least one sensor 130 is missing. For example, the processor 150 may detect whether data of the at least one sensor 130 among the plurality of sensors 130 used to generate information (e.g., exercise information or health information) associated with the user during the specified period is missing. According to an embodiment, the processor 150 may identify information associated with a session when the sensor 130 data is missing (hereinafter, a "missing session") (e.g., a start time, an end time, or a period of the missing session or a state of the user associated with the session (e.g., a sleep state, an exercise state, an activity state, and/or whether he/she is indoors/outdoors)).

According to an embodiment, the processor 150 may identify the measured sensor 130 data excluding the missing sensor 130 data during the specified period (e.g., the missing period). For example, the processor 150 may identify sensor 130 data measured by the other sensors 130 except for the sensor 130 corresponding to the missing sensor 130 data among the plurality of sensors 130.

According to an embodiment, the processor 150 may identify a measurement history of previously measured sensor 130 data. For example, the processor 150 may identify a measurement history of sensor 130 data measured during a previous period corresponding to the specified period (the missing session) (the session different from the missing session). According to an embodiment, the processor 150 may determine whether additional data is required to generate the missing sensor 130 data. For example, when the additional data is required to generate the missing sensor 130 data, the processor 130 may receive text data associated with behavior of the user during the specified period from the user. The processor 150 may obtain text data from the user through an input through the user interface, an input through the physical key, and/or a voice input through the microphone.

According to an embodiment, the processor 150 may generate the missing sensor 130 data based at least in part on the measured sensor 130 data, using the generative artificial intelligence model. According to an embodiment, the processor 150 may generate the missing biometric data based on sensor 130 data obtained through the other sensor 130 except for the sensor 130 corresponding to the missing sensor 130 data in the same session, sensor 130 data obtained in a different session, and/or the text data obtained from the user.

According to an embodiment, the processor 150 may obtain sensor 130 data measured using the at least one sensor 130. For example, the processor 150 may obtain sensor 130 data using the sensor 130 included in the electronic device 100 or may receive sensor 130 data from the external electronic device 100.

According to an embodiment, the processor 150 may set a parameter corresponding to a specified condition. For example, the processor 150 may set a parameter corresponding to a virtual health state.

According to an embodiment, the processor 150 may generate sensor 130 data under the specified condition based on the parameter and the sensor 130 data, using the generative artificial intelligence model. According to an embodiment, the processor 150 may apply the set parameter to the measured sensor 130 data to generate sensor 130 data under a specified virtual condition, using the generative artificial intelligence model. For example, the processor 150 may generate sensor 130 data corresponding to a virtual exercise state (e.g., anaerobic exercise, aerobic exercise, strength exercise, exercise with a lot of movement, or exercise with little movement), using the generative artificial intelligence model.

The case in which the electronic device 100 performs both the operations of measuring the data and generating the missing data is described in FIG. 1, but, according to various embodiments, the electronic device 100 may perform the operations of measuring the data and/or generating the missing data in conjunction with the external electronic device 100 and/or the external server. For example, the electronic device 100 may receive biometric data and/or sensor 130 data, which are/is obtained using the sensor 130 included in the external electronic device 100 (e.g., the wearable device), from the external electronic device 100. The electronic device 100 may generate missing data in at least in part conjunction with the external server (e.g., the generative artificial intelligence model of the external server).

According to an embodiment, the configurations of the electronic device 100 are limited to those illustrated in FIG. 1 and some configurations may be omitted or at least some configurations (e.g., the electronic device 110 and the wearable device 120 of FIG. 2 and/or at least one of the components of the electronic device 1501 of FIG. 15) may be added.

The electronic device 100 according to an embodiment of the disclosure may identify that biometric data and/or sensor data are/is missing during the specified cycle or the specified period and may generate the missing biometric data and/or sensor data, using the generative artificial intelligence model. According to an embodiment, the electronic device 100 may variously generate sensor data corresponding to a virtual condition to variously generate sensor data capable of being used to develop, analyze, and/or evaluate an algorithm even in a limited environment.

FIG. 2 is a block diagram of a system 200 according to an embodiment.

According to an embodiment, the system 200 may include an electronic device 210 (e.g., an electronic device 100 of FIG. 1 or an electronic device 1501 of FIG. 15), a wearable device 220 (e.g., an electronic device 1501, 1502, or 1504 of FIG. 15), and a server 230 (e.g., a server 1508 of FIG. 15).

According to an embodiment, the electronic device 210 may include an application 211 (e.g., an application 1546 of FIG. 15), an AI model 213 (e.g., an AI model 330 of FIG. 3, an AI model 430 of FIG. 4, or an AI model 930 of FIG. 9), and hardware 215 (e.g., hardware 350 of FIG. 3).

According to an embodiment, the application 211 may include a generative artificial intelligence-based application 211 (e.g., a health application) for providing analysis information associated with a user based on artificial intelligence. For example, the application 211 may provide the AI model 213 with at least one piece of sensor data and/or biometric data associated with the user. The sensor data and/or the biometric data may include sensor data and/or biometric data obtained through a sensor of the electronic device 210 and/or an external electronic device (e.g., the wearable device 220). For example, the application 211 may provide analysis information associated with the user through a user interface based on the data provided from the AI model 213. For example, the application 211 may provide the user with useful guide information, such as exercise, meditation, or counsel, based on biometric information.

According to an embodiment, the AI model 213 may include a generative artificial intelligence model. For example, the AI model 213 may include a model personalized in conjunction with a domain and may include a model used when processing data requiring security, such as biometric data of the user. For example, the AI model 213 may include an artificial intelligence model for collecting, training, analyzing, and/or predicting data regardless of a specific purpose and/or a domain. For example, the AI model 213 may include, but is not limited to, a large language model (LLM), a generative adversarial network, and/or a variational autoencoder. According to an embodiment, the AI model 213 may determine whether biometric data and/or sensor data are/is missing and may generate missing biometric data and/or missing sensor data based on a measurement history of previously obtained biometric data and/or sensor data, a user input (e.g., information indicating behavior of the user during a specified cycle or a specified period), and/or non-missing biometric data and/or sensor data.

According to an embodiment, the hardware 215 may include at least one processing device (e.g., a neural processing unit (NPU), a graphic processing unit (GPU), or a central processing unit (CPU)) and at least one hardware component (e.g., at least one sensor, communication circuitry, a memory, and/or a display). For example, the electronic device 210 may receive sensor data and/or biometric data from the wearable device 220 and may obtain biometric data of the user and/or sensor data using at least one sensor included in the electronic device 210. For example, the hardware 215 may include at least one of the components of the electronic device 1501 of FIG. 15.

According to an embodiment, the electronic device 210 may generate missing biometric data and/or missing sensor data, in conjunction with a cloud platform 233 of the server 230. For example, the electronic device 210 may transmit data (e.g., sensor data and/or biometric data) to the server 230 through communication circuitry (not shown) of the server 230 and may receive the result of processing data (e.g., missing biometric data and/or missing sensor data generated by the server 230) from the server 230. For example, the electronic device 210 may deliver the data received from the server 230 to the application 211.

According to an embodiment, the wearable device 220 may include an application 221 (e.g., an application 1546 of FIG. 15), a communication module 222 (e.g., a communication module 1590 of FIG. 15), a plurality of measurement modules, a hardware abstraction layer (HAL) 227, a plurality of sensors (e.g., a sensor module 1576 of FIG. 15), and storage 229 (e.g., a memory 1530 of FIG. 15).

According to an embodiment, the application 221 may provide information (e.g., activity information, health information, sleep information, and/or exercise information) associated with the user based on pieces of data provided from the plurality of measurement modules.

According to an embodiment, the communication module 222 may transmit and receive information and/or data with an external electronic device (e.g., the electronic device 210 and/or the server 230). For example, the communication module 222 may transmit biometric data obtained using the plurality of measurement modules and/or sensor data obtained using a plurality of sensors to the electronic device 210.

According to an embodiment, the plurality of measurement modules may generate biometric data associated with the user. The plurality of measurement modules may measure, track, monitor, and/or analyze the biometric data associated with the user. According to an embodiment, the plurality of measurement modules may include an action tracker 223-1, a stress tracker 223-2, a sleep tracker 223-3, a step tracker 223-4, a heart rate variability (HRV) module, and a mind tracker 223-6.

For example, the action tracker 223-1 may measure the number of actions per specified time (e.g., minute) of the user. For example, the action tracker 223-1 may track an action of the user, which does not have a specific pattern. The action tracker 223-1 may measure a degree, continuity, strength, and/or a displacement difference of the action of the user through a sensor (e.g., an inertial measurement unit 228-1).

The stress tracker 223-2 may determine stress of the user based on data provided from the HRV module 223-5. For example, the stress tracker 223-2 may determine or classify a stress index of the user. For example, the stress tracker 223-2 may classify the stress of the user as acute stress or chronic stress and may determine and provide a stress index. The stress tracker 223-2 may measure electrodermal activity (EDA) using a sensor (e.g., an electrode sensor 228-4) and may determine stress of the user based on tension of skin. The stress tracker 223-2 may synthetically analyze HRV information, IBI information, and various pieces of biometric information and may determine a stress index of the user and/or whether the user has acute/chronic stress.

The sleep tracker 223-3 may track a sleep state or a sleep pattern of the user. The sleep tracker 223-3 may measure a sleep posture of the user through a sensor (e.g., the inertial measurement unit 228-1). The sleep tracker 223-3 may measure a sleep posture of the user through a sensor (e.g., the inertial measurement unit 228-1). For example, the sleep tracker 223-3 may analyze a sleep state of the user based on data measured by the HRV module 223-5 during the sleep. For example, the sleep tracker 223-3 may extract sympathetic nerve and parasympathetic nerve elements through a frequency analysis for sensor data and may determine a sleep state of the user (e.g., an awake state, just before sleep, just after sleep, light sleep, deep sleep, or rapid eye movement (REM) sleep).

The step tracker 223-4 may measure (determine) a step count of the user based on an impulse value detected while the user is walking, which is measured using a sensor (e.g., an accelerometer sensor). For example, the step tracker 223-4 may identify an impulse value or a frequency of steps while the user is moving and may generate subdivided analysis information, such as a walking state (e.g., walking or running) of the user. For example, when identifying a step pattern of a specified value or more, the step tracker 223-4 may measure a step count of the user to increase the accuracy of measuring the step of the user. The step tracker 223-4 may provide information associated with a degree (speed) to which the user is walking or running, a time when the user moves, or a state of the user when he/she is walking or running, based on the measured information.

The HRV module 223-5 may measure regularity or variability of a heart rate of the user. For example, the HRV module 223-5 may measure a heart rate (HR) of the user. The HRV module 223-5 may identify information about a peak-to-peak inter-beat interval (IBI) of a series of pieces of heart rate information and may generate variance, deviation information, time series analysis information, and/or frequency analysis information from the IBI information.

The mind tracker 223-6 may determine a depression index of the user based on the biometric data collected from the plurality of measurement modules. The mind tracker 223-6 may determine the depression index based on information input from the user. The mind tracker 223-6 may track and observe the depression index of the user and may provide the user with a corresponding activity based on it.

According to various embodiments, the measurement modules included in the wearable device 220 are not limited to those illustrated in FIG. 2.

According to an embodiment, the HAL 227 may transmit and receive information and/or data between the application 221, the communication module 222, and/or the plurality of measurement modules and hardware (e.g., a plurality of sensors). For example, the HAL 227 may control operations of the plurality of sensors in conjunction with operations of the application 221, the communication module 222, and/or the plurality of measurement modules.

According to an embodiment, the plurality of sensors may include the inertial measurement unit 228-1, the GNSS sensor 228-2, the PPG sensor 228-3, and the electrode sensor 228-4.

For example, the inertial measurement unit 228-1 may include, but is not limited to, an accelerometer sensor, a gyro sensor, and/or a geomagnetic sensor. For example, the inertial measurement unit 228-1 may measure acceleration, a direction, a speed, a distance change, and/or a posture of the electronic device 210.

For example, the global navigation satellite system (GNSS) sensor may include a global positioning system (GPS) sensor, a GLONASS sensor, a BeiDou sensor, and/or a Galileo sensor. For example, the GNSS sensor 228-2 may measure a location of the electronic device 210.

For example, the photoplethysmography (PPG) sensor may emit light of various wavelengths and may measure biometric data (e.g., a heart rate and/or oxygen saturation) of the user based on light incident by being reflected from the body of the user. According to an embodiment, only the PPG sensor 228-3 is described in FIG. 2, but the wearable device 220 may include various optical sensors for biometric measurement including the PPG sensor 228-3. For example, the optical sensor may include at least one light source (e.g., a light-emitting element (e.g., an LED)) for emitting light of various wavelengths. For example, light of a green wavelength may be used to measure a heart rate and may have an advantage in which it relatively shallowly penetrates into the skin of the user to be robust to noise. For example, light of a red wavelength may relatively deeply penetrate into the skin of the user to be used to measure a heart rate with higher accuracy. For example, light of an infrared wavelength may be used to measure a heart rate and oxygen saturation (SpO2). Light of a blue wavelength may be used to measure blood glucose of the user. For example, the optical sensor may include a light detector including at least one photodiode.

For example, the electrode sensor 228-4 may include at least one electrode. For example, the electrode sensor 228-4 may include a sensor for measuring biometric data through the electrode. For example, the electrode sensor 228-4 may measure various pieces of biometric data in conjunction with another sensor (e.g., the PPG sensor 228-3).

According to various embodiments, the sensors included in the wearable device 220 are not limited to those illustrated in FIG. 2. For example, the wearable device 220 may further include a proximity sensor, a temperature (body temperature) sensor, a gas sensor, a fine dust sensor, a humidity sensor, an illumination sensor, a time-of-flight (TOF) sensor, and/or an ultra wideband (UWB) sensor (e.g., a LiDAR sensor).

The storage 229 may store pieces of data associated with an operation of the wearable device 220. For example, the storage 229 may store pieces of biometric data obtained through the plurality of measurement modules and/or pieces of sensor data obtained using the plurality of sensors.

According to an embodiment, the server 230 may include an AI model 231, a cloud platform 233, and hardware 235. According to an embodiment, the AI model 231 of the server 230 may correspond to the AI model 231 of the electronic device 210. For example, the server 230 (e.g., the AI model 231 of the server 230) may be configured to have fewer scale and spatial constraints than the electronic device 210 and support more amounts of computation using more resources to quickly and accurately process more amounts of data than the electronic device 210. According to an embodiment, the cloud platform 233 may be configured to transmit and receive data with the electronic device 210. According to an embodiment, the hardware 235 of the server 230 may include at least one processing device (e.g., a neural processing unit (NPU), a graphic processing unit (GPU), or a central processing unit (CPU)) and at least one hardware component (e.g., communication circuitry and/or a memory).

The system 200 according to an embodiment may identify that the biometric data and the sensor data are/is missing during the specified cycle or the specified period and may generate and use the missing biometric data and/or sensor data, using the generative artificial intelligence model.

FIG. 3 is a drawing illustrating a layer of a generative artificial intelligence model according to an embodiment. Hereinafter, the same or similar contents to the contents described in FIG. 2 will be omitted or briefly described.

According to an embodiment, an electronic device (e.g., an electronic device 100 of FIG. 1, an electronic device 120 of FIG. 2, or an electronic device 1501 of FIG. 15) may include a generative AI application 310 (e.g., an application 211 of FIG. 2), an AI model 330 (e.g., an AI model 213 of FIG. 2, an AI model 430 of FIG. 4, or an AI model 930 of FIG. 9), or hardware 350 (e.g., hardware 215 of FIG. 2).

According to an embodiment, the generative AI application 310 may include an application 310 (e.g., a health application 310) for providing analysis information associated with a user based on artificial intelligence. For example, the generative AI application 310 may provide the AI model 330 with at least one piece of sensor data and/or biometric data associated with the user. For example, the generative AI application 310 may provide analysis information associated with the user through a user interface based on the data provided from the AI model 330.

According to an embodiment, the AI model 330 may include a specified domain AI model 331 (e.g., a specific AI model 330) and a general AI model 333. For example, the AI model 330 may determine whether biometric data and/or sensor data are/is missing based on data (e.g., biometric data and/or sensor data) provided from the generative AI application 310 and may generate missing biometric data and/or missing sensor data.

According to an embodiment, the specified domain AI model 331 may include a specified domain (e.g., health)-based artificial intelligence model. For example, the specified domain AI model 331 may include a model personalized in conjunction with a domain and may include a model used when processing data requiring security, such as biometric data of the user.

For example, the general AI model 333 may include an artificial intelligence model for collecting, training, analyzing, and/or predicting data regardless of a specific purpose and/or a domain. The general AI model 333 may include an artificial intelligence model applicable to the entire data rather than a specific entity. For example, the general AI model 333 may include, but is not limited to, a large language model (LLM), a generative adversarial network, and/or a variational autoencoder.

According to an embodiment, the hardware 350 may include at least one processing device (e.g., a neural processing unit (NPU), a graphic processing unit (GPU), or a central processing unit (CPU)) and at least one hardware component (e.g., at least one sensor).

FIG. 4 is a drawing for describing an operation of generating missing biometric data in an electronic device according to an embodiment.

According to an embodiment, an electronic device (e.g., an electronic device 100 of FIG. 1, an electronic device 210 of FIG. 2, or an electronic device 1501 of FIG. 15) may generate missing biometric data (e.g., generative data 440) using an AI model based on measured biometric data (hereinafter referred to as "activity data/health data 410") and/or text data 420.

According to an embodiment, the electronic device may obtain the activity/health data 410 (e.g., the activity/health data may be referred to as "biometric data" in the disclosure) using a sensor of the electronic device and/or a sensor of an external electronic device (e.g., a wearable device). The activity/health data 410 may include data corresponding to a raw signal measured using at least one sensor and may include quantitative and/or qualitative data generated based on the raw signal. For example, the activity/health data 410 may include action tracking data, stress tracking data, sleep tracking data, step tracking data, HRV data, mind tracking data, location data, and a user profile. For example, the activity/health data 410 is not limited to those illustrated in FIG. 4. For example, the action tracking data may include information about an action (e.g., a degree, continuity, strength, and/or a displacement difference of an action) per specified time (e.g., minute) of the user. The stress tracking data may include information about a stress index and/or a stress type (e.g., acute stress or chronic stress) of the user. The sleep tracking data may include information about a sleep state, a sleep time, and/or a sleep pattern of the user. The step tracking data may include information about a walking pattern of the user, a step count of the user, a state in which the user walks or runs, and/or a movement speed of the user. The HRV data may include information associated with a heart rate of the user and/or regularity of a heartbeat. The mind tracking data may include information associated with a depression index of the user. The location data may include information about a location and/or a movement trajectory of the electronic device. The user profile may include information associated with personal information of the user (e.g., a health state, body information, a name, a gender, an age, a preference of the user, activity history, and/or device usage history).

According to an embodiment, the AI model 430 (e.g., an AI model 213 of FIG. 2, an AI model 330 of FIG. 3, or an AI model 930 of FIG. 9) may receive the activity/health data 410 and may generate missing data. For example, the specified domain AI model 431 (e.g., the health AI model 430) may generate or estimate new data based on data associated with a specified specific domain (e.g., a health domain).

According to an embodiment, the specified domain AI model 431 (e.g., a specified domain AI model 931 of FIG. 9) may estimate a new index based on the biometric data 410 (e.g., the activity/health data 410). For example, the specified domain AI model 431 may analyze a sleep type of the user based on accumulated sleep tracking data of the user or may measure a menstrual cycle of a female user based on accumulated skin temperature information and/or heart rate information of the user.

According to an embodiment, the specified domain AI model 431 may generate missing data. For example, when there is a day (hereinafter, a "missing day") when the activity/health data 410 associated with activity of the user is missing, due to deactivation of the sensor or malfunction of the sensor, and/or because the user does not have the electronic device or the external electronic device, the specified domain AI model 431 may generate missing activity/health data 410 based on a history of the activity/health data 410, which is measured on another day except for the missing day. According to an embodiment, when it is unable to generate the missing activity/health data 410 based on the activity/health data 410 measured on the other day except for the missing day or to increase accuracy when generating the missing activity/health data 410, the electronic device may receive the text data 420. For example, the text data 420 may include information associated with behavior of the user on the missing day. For example, it is assumed that the electronic device receives a voice input (a text input), "I went to the supermarket to buy groceries at lunchtime and went for a run around the park late at night.", from the user. The AI model 430 may generate a plurality of tokens based on a semantic unit of text, such as the text input, "went to-supermarket-buy-groceries-at-lunchtime-went-run-around-park-late-night". For example, the AI model 430 may generate the plurality of tokens from the voice input (the text input based on a semantic unit of each language depending on the language (e.g., a language for each country). For example, for an English voice input (text input), the AI model 430 may generate each word of the voice input (text input) as a token. For example, the AI model 430 may generate the plurality of tokens based on different semantic units depending on a scale, a characteristic, a training scheme, training data, and/or a type of the AI model 430. For example, the AI model 430 (e.g., the specified domain AI model 431) may perform prompting to output data in the same form as the activity/health data 410 previously recorded with the general AI model 433 (e.g., a general AI model 933 of FIG. 9) based on the token from the text input. For example, the general AI model 433 may output data in the same form as the previously recorded activity/health data 410 based on the tokens. For example, the general AI model 433 may extract (or infer) main keyword information from tokens, such as Lunch is from 11:00 to 13:00", "Mart is location information", "late at night is after 22:00", and "Running is movement at a speed of 4 km/h or more". The general AI model 433 may perform provision (e.g., inferring) of the extracted (or inferred) information to the specified domain AI model 431. The specified domain AI model 431 may determine similarity with the existing activity/health data 410 based on the information provided from the general AI model 433 and may generate missing activity/health data 410 based on it. For example, when the missing day is yesterday, the specified domain AI model 431 may extract data and/or information estimated as being similar to yesterday and necessary to generate missing day based on the activity/health data 410 except for the missing day and/or the information received from the general AI model 433 and may generate missing activity/health data based on the extracted data and/or information.

According to an embodiment, when there is no or a lack of sensor data for generating the activity/health data 410, the AI model 430 (e.g., the specified domain AI model 431) may generate required sensor data. For example, when measuring daily calorie consumption of the user, a value obtained by calculating the calorie consumption may vary depending on a movement speed, a gradient (e.g., an uphill/downhill), or activity end (e.g., walking or running). For example, even when the same step count is measured, calorie consumption may more increase for an uphill than a downhill. In this case, the AI model 430 may generate altitude data estimated based on pedometer information for measuring a step count of the user and location information of the electronic device (e.g., a mountain). When calculating daily calorie consumption, the AI model 430 may additionally consider the generated altitude data to calculate and/or modify the calorie consumption.

According to an embodiment, a step count, an activity time, and calories burned are illustrated as the generative data 440 in FIG. 4, but the generative data 440 is not limited thereto. Even any of the missing biometric data 410 (e.g., the activity/health data 410) is possible as the generative data 440.

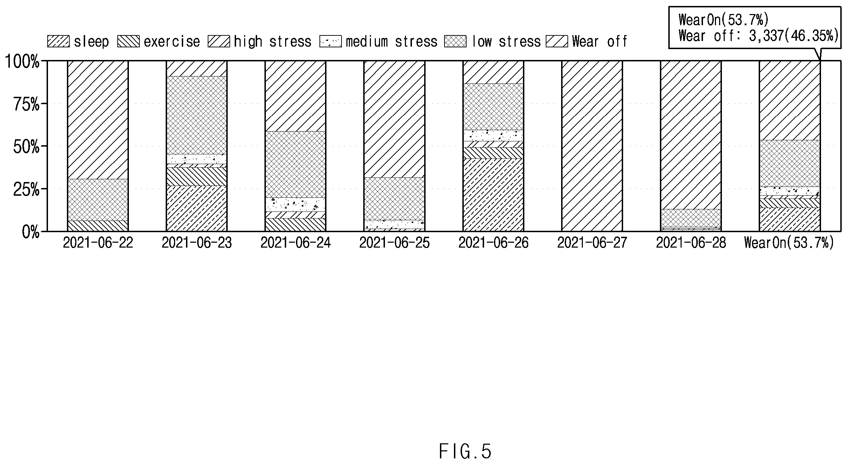

FIG. 5 is a drawing for describing an operation of detecting that data is missing in an electronic device according to an embodiment. For example, a description will be given in FIG. 5 assuming that an electronic device (e.g., an electronic device 100 of FIG. 1, an electronic device 210 of FIG. 2, or an electronic device 1501 of FIG. 15) obtains biometric data and/or sensor data from an external electronic device (e.g., a wearable device (e.g., a wearable device 220 of FIG. 2)).

According to an embodiment, the electronic device may identify biometric information measured for each specified cycle (e.g., it is assumed that it is a daily cycle, but not limited thereto). For example, FIG. 5 is a graph illustrating day-to-day biometric information (e.g., sleep, exercise, high stress, medium stress, low stress, and wear off) identified by the electronic device. According to various embodiments, pieces of biometric information are not limited to those illustrated and described in FIG. 5. For example, the electronic device may determine whether biometric data is missing based on a ratio of wear off for each specified period (e.g., day). For example, when the time when the external electronic device is not worn exceeds a specified threshold time, the electronic device may determine that the biometric data is missing during a corresponding period. For example, when obtaining biometric data from the external electronic device, the electronic device may fail to obtain the biometric data during a state (time) in which the user does not wear the external electronic device. For example, when the time when the external electronic device is not worn during a specified period (e.g., one day (24 hours)) exceeds the specified threshold time (e.g., 8 hours) or a specified threshold ratio (e.g., about 70%), the electronic device may determine that the biometric data is missing on the corresponding date. Referring to FIG. 5, for example, assuming that the specified threshold time is 12 hours (i.e., the threshold ratio is 50%), the electronic device may determine that the biometric data is missing on 2021-06-22, 2021-06-25, 2021-06-27, and 2021-06-28.

According to an embodiment, the electronic device may compare a time when the biometric data is missing with the specified threshold time based on at least in part on an amount of a time when the biometric data (or sensor data) is measured or an operation state of the electronic device and may determine that the biometric data (or sensor data) is missing when the time exceeds the specified threshold time. For example, when a ratio between a time when the biometric data (or sensor data) is not obtained for each specified cycle (or specified session) or a time when the biometric data (or sensor data) is not obtained in the specified period (or session) exceeds the specified threshold, the electronic device may determine that the biometric data (or sensor data) is missing in the corresponding period (or session).

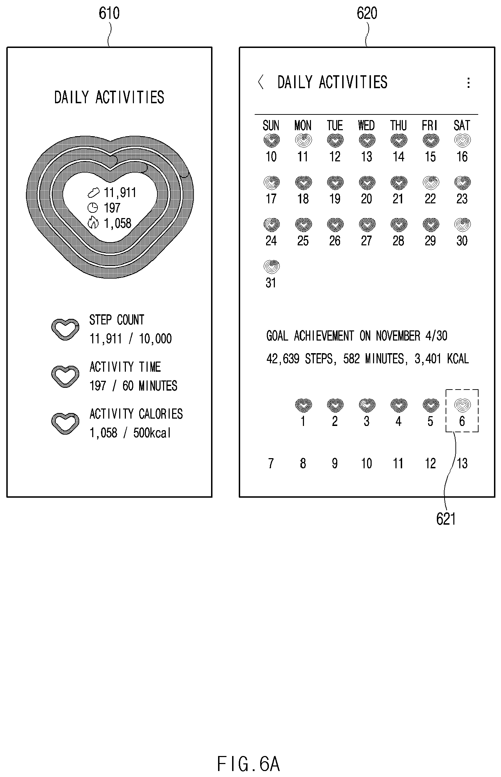

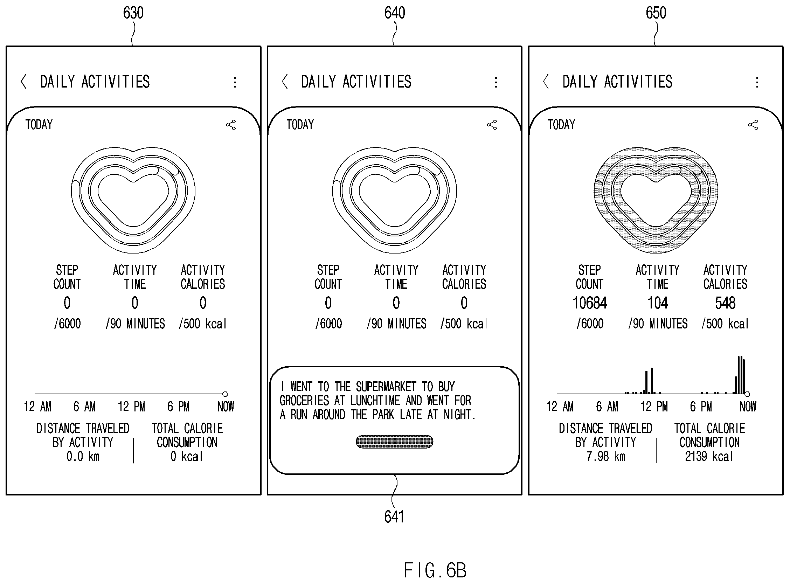

FIGS. 6A and 6B are drawings for describing an operation of generating and providing missing data in an electronic device according to an embodiment.

According to an embodiment, an electronic device (e.g., an electronic device 100 of FIG. 1, an electronic device 210 of FIG. 2, or an electronic device 1501 of FIG. 15) may provide a user interface including measured biometric data (e.g., activity/health data 410 of FIG. 2) and information indicating whether the biometric data is missing. For example, a description will be given in FIGS. 6A and 6B assuming that a specified period is a daily unit, but not limited thereto.

For example, a first screen 610 indicates a screen which displays biometric information of a user in a current cycle (e.g., today). For example, the first screen 610 may include an image and/or information indicating an amount of daily activities of the user. For example, the first screen 610 may include information indicating a "step count/target step count", an "activity time/target activity time", and "activity calories/target calorie consumption" of the user and a visual element indicating whether a target value is achieved.

For example, a second screen 620 is a screen summarizing and indicating biometric information of the user, which is measured for each cycle. For example, the second screen 620 may summarize and provide biometric information for each specified cycle (e.g., day) for a period (e.g., a month) longer than the specified cycle. For example, the second screen 620 may include a visual element (e.g., a figure) 621 indicating a degree to which biometric information for each cycle is measured. For example, the first figure 621 may have a form, the inside of which is not filled to indicate that biometric information is missing on a corresponding date. For example, FIG. 6B illustrates indicating a degree to which biometric information is measured according to a degree to which the inside of a figure is filled with another color or that the biometric information is missing, but a display method is not limited thereto. For example, the visual element may be set to indicate a degree to which biometric information is measured according to a color, a size, a shape, and/or contrast or that the biometric information is missing.

For example, a third screen 630 indicates a screen which displays biometric information on a date when biometric data is missing. For example, the electronic device may receive a user input for selecting a visual element (e.g., a first figure) indicating that biometric data is missing through a user interface. The electronic device may display the third screen 630 including biometric information during a cycle (date) selected according to the user input. For example, because the biometric data is missing, all of values indicating the biometric data on the third screen 630 may be set to an initial value.

For example, the electronic device may output a message for requesting information associated with behavior of the user during the period (date) when the biometric data is missing from the user through the user interface, in response to outputting the third screen 630.

For example, a fourth screen 640 indicates a screen which displays contents of a user input on one region 641, when the electronic device receives the user input including information associated with behavior of a user during a cycle (date) when biometric data is missing. For example, the one region 641 of the user interface may display text contents identified from the user input. For example, the user input may include a voice input and/or an input received through the user interface.

According to an embodiment, a fifth screen 650 indicates a screen on which the electronic device generates and displays biometric data during the period (date), using a generative artificial intelligence model (e.g., an AI model 213 of FIG. 2, an AI model 330 of FIG. 3, an AI model 430 of FIG. 4, or an AI model 930 of FIG. 9). For example, the electronic device may generate missing biometric data based on biometric data obtained on a day excluding the missing day and/or the user input, using the generative artificial intelligence model. The fifth screen 650 may include information and a visual element indicating the generated biometric data. The fifth screen 650 may include information and/or a diagram analyzed using the generated biometric data.

According to various embodiments, the configuration of the user interface provided by the electronic device and the information and/or the visual element included in the user interface are not limited to those illustrated in FIG. 6.

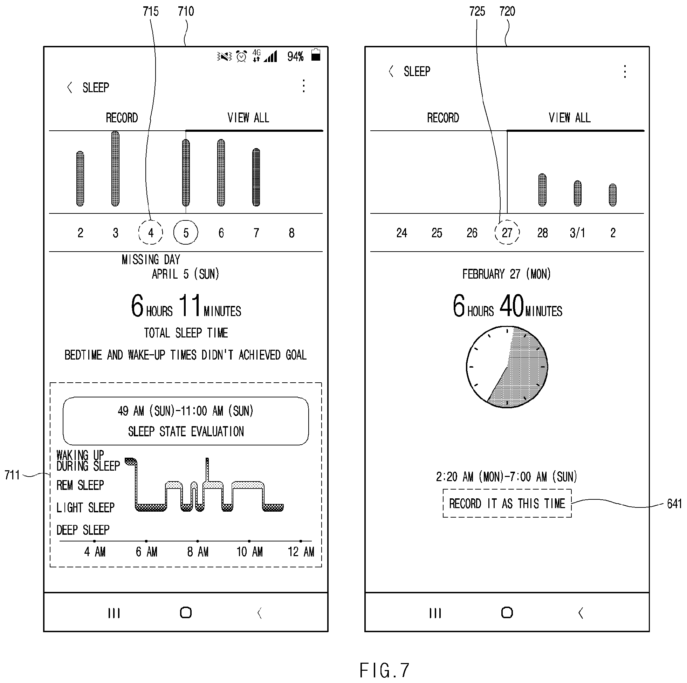

FIG. 7 is a drawing for describing an operation of generating and providing missing data in an electronic device according to an embodiment. For example, FIG. 7 illustrates an example of a user interface screen for providing sleep data among pieces of biometric data.

According to an embodiment, an electronic device (e.g., an electronic device 100 of FIG. 1, an electronic device 210 of FIG. 2, or an electronic device 1501 of FIG. 15) may provide biometric data through a user interface. A first screen 710 of the user interface may include a graph indicating sleep data measured for each day and detailed information of the sleep data for each day (e.g., a total sleep time and information obtained by analyzing a sleep state). For example, the first screen 710 may indicate that sleep data is missing (715) in a specific cycle (date) (e.g., April 4) through the graph indicating the sleep data measured for each day. For example, the electronic device may generate and provide missing sleep data based on biometric data measured on a date excluding the missing day (e.g., April 4) and/or the user input, using a generative artificial intelligence model (e.g., an AI model 213 of FIG. 2, an AI model 330 of FIG. 3, an AI model 430 of FIG. 4, or an AI model 930 of FIG. 9).

According to an embodiment, a second screen 720 indicates a screen on which the user interface provides an item for generating missing sleep data corresponding to a missing day (February 27) and allowing a user to confirm information of the generated sleep data. For example, the second screen 720 may indicate that sleep data is missing (725) in a specific cycle (date) (e.g., February 27) through the graph indicating the sleep data measured for each day. For example, the second screen 720 may provide the information of the generated sleep data (e.g., a time when the user is asleep, a time when the user awakes, and a sleep time) and may receive an input for confirming whether to store the generated biometric data from the user through the item 641. For example, when receiving the input for storing the generated biometric data from the user, the electronic device may store the generated biometric data. When not receiving an input for storing generated missing biometric data from the user, the electronic device may store the generated missing biometric data.

According to various embodiments, the configuration of the user interface provided by the electronic device and the information and/or the visual element included in the user interface are not limited to those illustrated in FIG. 7.

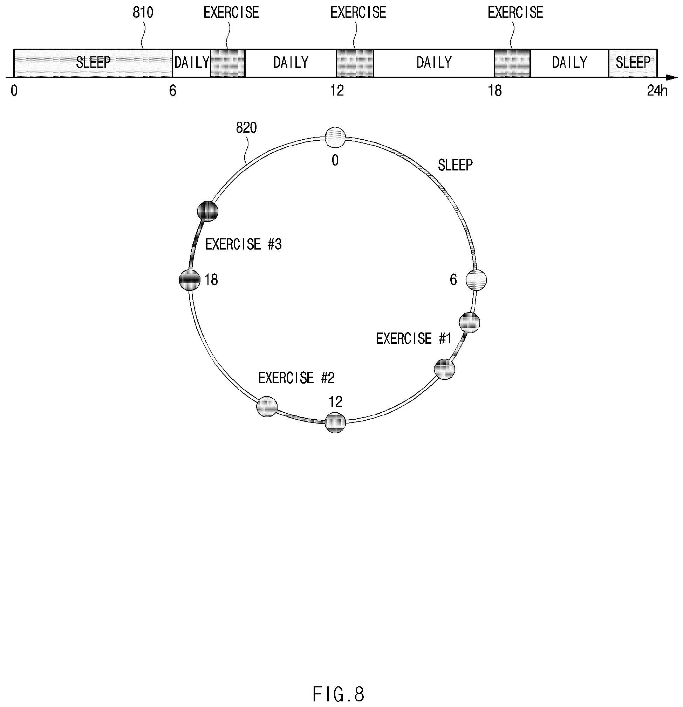

FIG. 8 illustrates an example of a user interface provided to manage data by an electronic device according to an embodiment.

According to an embodiment, the electronic device may provide interfaces 810 and 820 for modifying information of biometric data corresponding to one cycle (e.g., a specific date) and the biometric data.

For example, the interfaces 810 and 820 may provide the information of the biometric data in the form of a specified graph. For example, FIG. 8 illustrates a case in which the interfaces 810 and 820 include a graph indicating an activity state (e.g., sleep, daily life, or exercise) of a user during one cycle (one day) of the user. For example, the interfaces may include the first graph 810 indicating a daily activity state of the user and the second graph 820 configured to adjust a time of each activity state through a user input. For example, the second graph 820 may be provided in the form of a circular graph capable of selecting start and end points of each activity state of the user for each day. For example, the user may select a point indicating the start or end point of each activity state and may input a gesture (e.g., a drag input in a specific direction) to adjust a start or end time of each activity state. According to an embodiment, when a portion of biometric data is modified by the user input, the electronic device may store the modified biometric data.

FIG. 8 illustrates an example of an interface capable of displaying and adjusting biometric data. The interfaces provided by the electronic device are not limited to those illustrated in FIG. 8.

FIG. 9 is a drawing for describing an operation of generating missing sensor data in an electronic device according to an embodiment. Hereinafter, the same or similar description to the description of FIG. 4 will be omitted or briefly described.

According to an embodiment, an electronic device (e.g., an electronic device 100 of FIG. 1, an electronic device 210 of FIG. 2, or an electronic device 1501 of FIG. 15) may generate missing data (generative data) 940 using an AI model (e.g., an AI model 213 of FIG. 2, an AI model 330 of FIG. 3, or an AI model 430 of FIG. 4) based on measured sensor data 910 and/or text data 920.

According to an embodiment, the electronic device may obtain the sensor data 910 using a sensor of the electronic device and/or a sensor of an external electronic device (e.g., a wearable device). The sensor data 910 may include data corresponding to a raw signal measured using at least one sensor. For example, the sensor data 910 may include electrocardiogram (EEG) data, galvanic skin reflect (GSR) data, electroencephalogram (EEG) data, bioelectrical impedance analysis (BIA) data, photoplethysmography (PPG) data, accelerometer (ACC) data, gyro data, temperature data, time of flight (TOF) data, ultra-wideband (UWB) data, GPS data, pedometer data, barometer data, and user profile. For example, the sensor data 910 is not limited to those illustrated in FIG. 9.

According to an embodiment, the AI model 930 may receive the sensor data 910 to generate missing data (generative data 940). According to an embodiment, a specified domain AI model 931 may generate or estimate new data based on data associated with a specified specific domain (e.g., a health domain).

According to an embodiment, the specified domain AI model 931 may estimate or generate the new sensor data 910 based on the measured sensor data 910. For example, the specified domain AI model 931 may generate missing data. For example, when there is a session (hereinafter, a "missing session") in which the sensor data 910 is missing, due to deactivation of at least one sensor or a malfunction of the at least one sensor, and/or because the user does not have the electronic device or the external electronic device, the specified domain AI model 931 may generate missing sensor data 940 (the generative data 940) based on a history of the sensor data 910, which is measured using another sensor except for the sensor corresponding to the missing sensor data 940 during the missing session. The specified domain AI model 931 may generate the missing sensor data 940 (the generative data 940) based on a history of the sensor data 910, which is measured during another session different from the missing session. For example, the specified domain AI model 931 may generate the missing sensor data 940 based on the sensor data 910 measured from a sensor except for a first sensor corresponding to the missing sensor data 940 in the same session (the missing session) and/or the sensor data 910 measured from the first sensor or another sensor, which is measured in a different session.

According to an embodiment, when it is unable to generate the missing sensor data 940 based on the sensor data 910 measured using another sensor during the missing session and/or the sensor data 910 measured during another session except for the missing session or to increase accuracy when generating the missing sensor data 940, the electronic device may receive text data 920 from the user. For example, the text data 920 may include information associated with behavior of the user in the missing session. For example, it is assumed that the electronic device receives a voice input (a text input), "I ran 400m track 5 laps at a pace of 1 minute and 30 seconds". The AI model 930 may generate a plurality of tokens based on a semantic unit of text, such as the text input, "ran-400-m-track-5-laps-at-pace-1 minute-30 seconds". For example, the AI model 930 (e.g., the specified domain AI model 931) may perform prompting to output data in the same form as the sensor data 910 previously recorded with a general AI model 933 based on the token from the text input. For example, the general AI model 933 may output data in the same form as the previously recorded sensor data 910 based on the tokens. For example, the general AI model 933 may extract (or infer) main keyword information from the token. The general AI model 933 may perform provision (e.g., inferring) of the extracted (or inferred) information to the specified domain AI model 931. The specified domain AI model 931 may determine similarity with the existing sensor data 910 based on the information provided from the general AI model 933 and may generate the missing sensor data 940 based on it.

According to an embodiment, PPG data, ACC data, atmospheric pressure data, and gyro data are illustrated as the generative data 940 in FIG. 9, but the generative data 940 is not limited thereto. Even any of the missing sensor data 940 is possible as the generative data 940.

For example, the electronic device (e.g., a mobile terminal) may obtain biometric data and/or sensor data of the user using at least one sensor or may obtain biometric data and/or sensor data of the user, which are/is measured by the external electronic device (e.g., the wearable device) from the external electronic device. There is a need for the user to always carry or wear the electronic device or the external electronic device such that the electronic device obtains biometric data and/or sensor data of the user. For example, when the user does not continue carrying or wearing the electronic device or the external electronic device, biometric data and/or sensor data may be missing. In this case, biometric information and/or health information of the user may fail to be accurately provided or bias may occur and an error may occur in statistics of data. Furthermore, it may take a lot of time and effort to obtain biometric data and/or sensor data sufficient to analyze a health state of the user. According to embodiments of the disclosure, an electronic device capable of generating missing biometric data and/or sensor data using a generative artificial intelligence model to supplement required data even when biometric data and/or sensor data are/is missing, a method, and a storage medium are provided.

An electronic device according to an embodiment of the disclosure may include communication circuitry, a display, at least one sensor, a memory including a generative artificial intelligence model, and a processor. According to an embodiment, the memory may store instructions that, when executed by the processor, control an operation of the electronic device.

According to an embodiment, the instructions, when executed by the processor, may cause the electronic device to identify biometric data periodically measured using the at least one sensor.

According to an embodiment, the instructions, when executed by the processor, may cause the electronic device to determine whether the biometric data is missing.

According to an embodiment, the memory, when executed by the processor, may cause the electronic device to identify information related to a period during which the biometric data was missing.

According to an embodiment, the instructions, when executed by the processor, may cause the electronic device to identify a measurement history of previously measured biometric data.

According to an embodiment, the instructions, when executed by the processor, may cause the electronic device to, using the generative artificial intelligence model, generate missing biometric data based at least in part on the measurement history.

According to an embodiment, the instructions, when executed by the processor, may cause the electronic device to receive text data associated with behavior of a user during the period.

According to an embodiment, the instructions, when executed by the processor, may cause the electronic device to generate the missing data based on the text data and the measurement history.

According to an embodiment, the at least one sensor may include a plurality of sensors.

According to an embodiment, the instructions, when executed by the processor, may cause the electronic device to identify sensor data measured using the plurality of sensors during a specified period.

According to an embodiment, the instructions, when executed by the processor, may cause the electronic device to detect whether sensor data corresponding to at least one sensor among the plurality of sensors is missing.

According to an embodiment, the instructions, when executed by the processor, may cause the electronic device to identify measured sensor data excluding missing sensor data during the specified period.

According to an embodiment, the instructions, when executed by the processor, may cause the electronic device to, using the generative artificial intelligence model, generate the missing sensor data based at least in part on the measured sensor data.

According to an embodiment, the instructions, when executed by the processor, may cause the electronic device to identify a measurement history of sensor data measured in a previous measurement period corresponding to a specified period.

According to an embodiment, the instructions, when executed by the processor, may cause the electronic device to generate the sensor data based at least in part on the measurement history.

According to an embodiment, the instructions, when executed by the processor, may cause the electronic device to receive text data associated with behavior of a user during the specified period from the user.

According to an embodiment, the instructions, when executed by the processor, may cause the electronic device to generate the sensor data based at least in part on the text data.

According to an embodiment, the instructions, when executed by the processor, may cause the electronic device to obtain at least some of biometric data or sensor data obtained through a sensor of an external electronic device from the external electronic device.

According to an embodiment, the instructions, when executed by the processor, may cause the electronic device to determine whether data is missing for the obtained at least some of the biometric data or the sensor data.

According to an embodiment, the instructions, when executed by the processor, may cause the electronic device to, using the generative artificial intelligence model, generate missing data for the at least some of the biometric data or the sensor data.

According to an embodiment, the instructions, when executed by the processor, may cause the electronic device to compare, for each specified cycle, a time when the biometric data is missing with a specified threshold time based on at least one of an amount of a time when the biometric data is measured or an operation state of the electronic device.

According to an embodiment, the instructions, when executed by the processor, may cause the electronic device to, when the time exceeds the specified threshold time, determine that the biometric data is missing.

According to an embodiment, the instructions, when executed by the processor, may cause the electronic device to output, through the display, a user interface including the measured biometric data and information indicating whether the biometric data is missing.

According to an embodiment, the instructions, when executed by the processor, may cause the electronic device to, when the missing biometric data is generated, receive a user input confirming whether to use the generated biometric data through the user interface.

According to an embodiment, the instructions, when executed by the processor, may cause the electronic device to store the generated biometric data in the memory based on the user input.

According to an embodiment, the instructions, when executed by the processor, may cause the electronic device to receive a user input for modifying the generated missing biometric data or the missing sensor data.

According to an embodiment, the instructions, when executed by the processor, may cause the electronic device to store the modified missing biometric data or missing sensor data in the memory based on the user input.

According to an embodiment, the instructions, when executed by the processor, may cause the electronic device to obtain sensor data measured using the at least one sensor.

According to an embodiment, the instructions, when executed by the processor, may cause the electronic device to set a parameter corresponding to a specified condition.

According to an embodiment, the instructions, when executed by the processor, may cause the electronic device to, using the generative artificial intelligence model, generate sensor data under the specified condition based on the parameter and the obtained sensor data.

According to embodiments of the disclosure, it may be identified that the biometric data and/or the sensor data are/is missing during the specified cycle or the specified period and the missing biometric data and/or sensor data may be generated and used, using the generative artificial intelligence model. According to embodiments of the disclosure, sensor data corresponding to a virtual condition may be variously generated to variously generate sensor data capable of being used to develop, analyze, and/or evaluate an algorithm even in a limited environment.

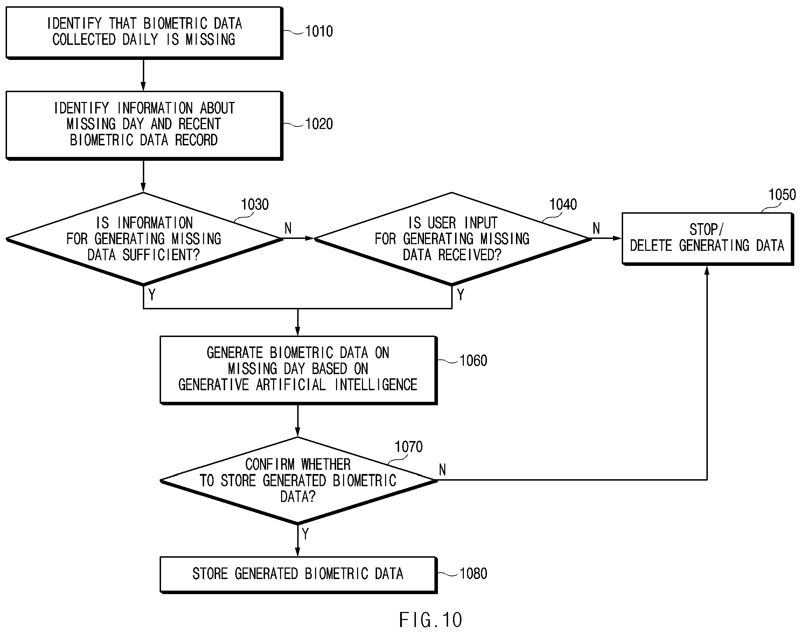

FIG. 10 is a flowchart of a method for managing data of an electronic device according to an embodiment.

In an embodiment below, the respective operations may be sequentially performed, but are not necessarily sequentially performed. For example, an order of the respective operations may be changed and at least two operations may be performed in parallel.

According to an embodiment, operations 1010 to 1080 may be understood as being performed by a processor (e.g., a processor 150 of FIG. 1) of an electronic device (e.g., an electronic device 100 of FIG. 1).

According to an embodiment, in operation 1010, an electronic device (e.g., an electronic device 100 of FIG. 1, an electronic device 210 of FIG. 2, or an electronic device 1501 of FIG. 15) may identify that biometric data collected daily is missing. For example, the electronic device may identify a day when biometric data is not collected (measured). According to an embodiment, the electronic device may receive biometric data obtained by an external electronic device from the external electronic device. The electronic device may identify whether at least a portion of the biometric data collected daily is missing based on the biometric data received from the external electronic device. According to an embodiment, the electronic device may determine whether biometric data is missing based on information indicating a state in which the external electronic device (e.g., a wearable device) is worn. For example, the electronic device may determine whether the biometric data is missing based on information about a time when the user wears the external electronic device or a time when the user does not wear the external electronic device. For example, the electronic device may determine whether the time when the user daily wears the external electronic device is less than a specified threshold time and may determine a day when the time when the external electronic device is worn is less than the threshold time as a day when biometric data is missing. In FIG. 10, the description is given assuming that the cycle is one day, but not limited thereto. The cycle may be specified as various periods. For example, the electronic device may identify that biometric data collected weekly is missing or may identify that biometric data collected monthly is missing.

According to an embodiment, in operation 1020, the electronic device may identify information about a missing day and a recent biometric data record. For example, the electronic device may identify the missing day and the information associated with the missing day (e.g., weather, a day of the week, a date (a day, a month, and/or a year), and/or schedule information of the corresponding date). The electronic device may identify a record of biometric data measured before and after the missing day.

According to an embodiment, in operation 1030, the electronic device may determine whether information for generating missing biometric data is sufficient. For example, the electronic device may determine whether it is able to generate missing biometric data based on the information about the missing day and the recent biometric data record, which are identified in operation 1020. According to an embodiment, the electronic device may perform operation 1060, when the information for generating the missing data is sufficient, and may perform operation 1040, when the information is not sufficient.

According to an embodiment, in operation 1040, the electronic device may identify whether a user input for generating missing data is received. According to an embodiment, the electronic device may output a message for requesting the user input to generate the missing data. For example, the electronic device may output a message for confirming whether there is specific behavior of the user on the missing day, such as "Did you go to work on the missing day?" or "Did you do your usual exercise from 8 to 10 on the missing day?". According to an embodiment, the user input may include information associated with behavior performed by the user on the missing day. For example, the user input may include a text input associated with the behavior of the user on the missing day. For example, the electronic device may receive a text input associated with the behavior of the missing day, such as "I woke up at 6:00 on the missing day, went to work for 30 minutes starting at 8:00, took a walk for about 30 minutes at 12:00, got off work at 6:00, and went to sleep around 10:00.", from the user. For example, the text input may include a voice input of the user and/or an input through a user interface.

According to an embodiment, the electronic device may perform operation 1060, when receiving the user input for generating the missing data, and may perform operation 1050, when not receiving the user input.

According to an embodiment, in operation 1050, the electronic device may stop or delete generating the missing data. For example, when the information for generating the missing data is not sufficient and not receiving the user input, the electronic device may stop generating the missing data and may provide a notification indicating that it is unable to generate the missing data. For example, when the user confirms not to store the generated missing biometric data, the electronic device may delete the generated missing biometric data.

According to an embodiment, in operation 1060, the electronic device may generate biometric data on the missing day based on a generative artificial intelligence model (e.g., an AI model 213 of FIG. 2, an AI model 330 of FIG. 3, an AI model 430 of FIG. 4, or an AI model 930 of FIG. 9). For example, the electronic device may generate missing biometric data based on the recent biometric data record and/or the user input, using the generative artificial intelligence model. For example, the electronic device may generate missing biometric data based on biometric data previously measured on a date of an environment similar to the missing day and/or information associated with behavior of the user on the missing day.

According to an embodiment, in operation 1070, the electronic device may confirm whether to store the generated missing biometric data. For example, the electronic device may provide a user interface for confirming whether to use the generated missing biometric data. The electronic device may receive a user input for determining whether to use (or store) the generated missing biometric data through the user interface. According to an embodiment, the electronic device may perform operation 1080, when receiving the user input for determining to store the generated missing biometric data, and may perform operation 1050, when receiving the user input for determining not to store the generated missing biometric data.

According to an embodiment, in operation 1080, the electronic device may store the generated missing biometric data in a memory.

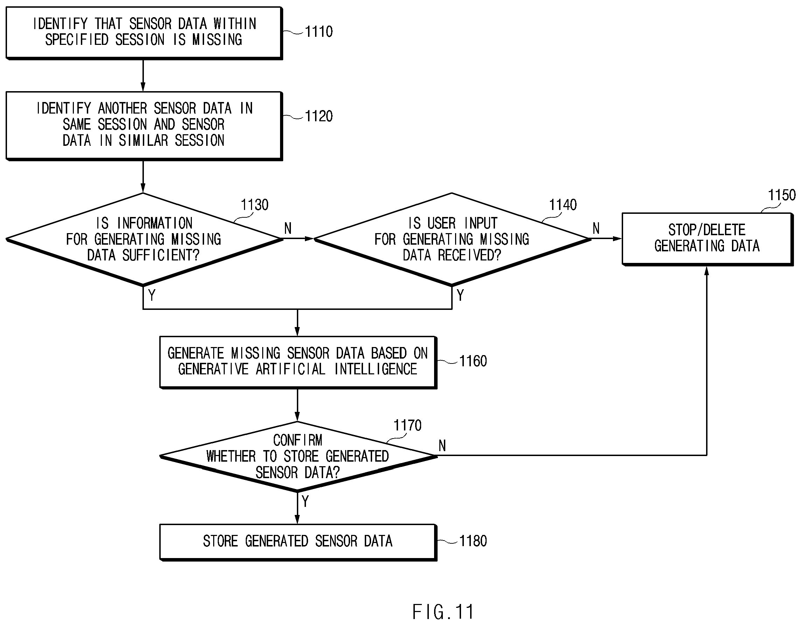

According to various embodiments, an order of the operations of FIG. 10 may be changed and at least some of the operations may be omitted and/or at least one operation (e.g., at least one of the operations of FIGS. 11 to 14) may be added.

According to an embodiment of the disclosure, it may be identified that the biometric data is missing during the specified cycle or the missing biometric data may be generated and used, using the generative artificial intelligence model.

FIG. 11 is a flowchart of a method for managing data of an electronic device according to an embodiment.

In an embodiment below, the respective operations may be sequentially performed, but are not necessarily sequentially performed. For example, an order of the respective operations may be changed and at least two operations may be performed in parallel.

According to an embodiment, operations 1110 to 1180 may be understood as being performed by a processor (e.g., a processor 150 of FIG. 1) of an electronic device (e.g., an electronic device 100 of FIG. 1).

According to an embodiment, in operation 1110, an electronic device (e.g., an electronic device 100 of FIG. 1, an electronic device 210 of FIG. 2, or an electronic device 1501 of FIG. 15) may identify that sensor data within a specified session is missing. For example, the electronic device may detect a case in which at least one sensor is deactivated within the specified session and sensor data is not obtained. For example, when obtaining sensor data using a plurality of sensors, the electronic device may identify a case in which sensor data corresponding to the at least one sensor is not measured. According to an embodiment, the plurality of sensors may include at least one sensor included in the electronic device and/or at least one sensor included in an external electronic device. For example, the electronic device may detect a case in which quality of specific sensor data is not good or accurate or a case in which it is determined that there is an error in sensor data (e.g., a case in which the sensor data is out of a specified range), as well as a case in which the sensor data is not collected at all. According to an embodiment, a session may refer to a time during a certain period specified by the electronic device.

According to an embodiment, in operation 1120, the electronic device may identify another sensor data in the same session and sensor data in a similar session. For example, the electronic device may identify sensor data obtained using another sensor except for a sensor corresponding to the missing sensor data. The electronic device may determine a previous session similar to a session when sensor data is missing based on session-related information (e.g., a period, a date, a time zone, a day of the week, weather, and/or a season). The electronic device may identify sensor data measured in the similar previous session.

According to an embodiment, in operation 1130, the electronic device may determine whether information for generating missing data is sufficient. For example, the electronic device may determine whether it is able to generate missing sensor data based on the other sensor data in the same session and the sensor data in the similar session, which are identified in operation 1120. According to an embodiment, the electronic device may perform operation 1160, when the information for generating the missing data is sufficient, and may perform operation 1140, when the information is not sufficient.

According to an embodiment, in operation 1140, the electronic device may identify whether a user input for generating missing data is received. According to an embodiment, the electronic device may output a message for requesting the user input to generate the missing data. According to an embodiment, the electronic device may receive a user input including information associated with behavior of the user within the session from the user. For example, the user input may include a text input associated with the behavior of the user during the session. For example, the electronic device may receive a text input, such as "I ran 400m track 5 laps at a pace of 1 minute and 30 seconds." or "I felt my heart pounding while eating dinner.". For example, the text input may include a voice input of the user and/or an input through a user interface.

According to an embodiment, the electronic device may perform operation 1160, when receiving the user input for generating the missing data, and may perform operation 1150, when not receiving the user input.

According to an embodiment, in operation 1150, the electronic device may stop or delete generating the missing data. For example, when the information for generating the missing data is not sufficient and not receiving the user input, the electronic device may stop generating the missing data and may provide a notification indicating that it is unable to generate the missing data. For example, when the user confirms not to store the generated missing sensor data, the electronic device may delete the generated missing sensor data.

According to an embodiment, in operation 1160, the electronic device may generate missing sensor data within the session based on a generative artificial intelligence model (e.g., an AI model 213 of FIG. 2, an AI model 330 of FIG. 3, an AI model 430 of FIG. 4, or an AI model 930 of FIG. 9). For example, the electronic device may generate missing sensor data based on the other sensor data in the same session, the sensor data in the similar session, and/or the user input, using the generative artificial intelligence model. For example, it is assumed that the electronic device obtains sensor data using a PPG sensor (e.g., heart rate measurement), a barometer (e.g., altitude measurement), and a pedometer (e.g., average speed measurement) to measure an exercise state of the user in a specific session. For example, when detecting that sensor data of one of the PPG sensor, the barometer, or the pedometer is missing in the same session, the electronic device may generate missing sensor data based on sensor data obtained using the remaining two sensors in the same session, using the generative artificial intelligence model. For example, when there is sensor data measured using the PPG sensor, the barometer, and/or the pedometer in another similar session, the electronic device may generate missing sensor data based on sensor data in the other similar session. For example, the electronic device may generate missing sensor data based on sensor data measured in a situation (or session) associated with behavior of the user, based on the user input including the information associated with the behavior of the user in the corresponding session.

According to an embodiment, in operation 1170, the electronic device may confirm whether to store the generated missing sensor data. For example, the electronic device may provide a user interface for confirming whether to use the generated missing sensor data. The electronic device may receive a user input for determining whether to use (or store) the generated missing sensor data through the user interface. According to an embodiment, the electronic device may perform operation 1180, when receiving the user input for determining to store the generated missing sensor data, and may perform operation 1150, when receiving the user input for determining not to store the generated missing sensor data.

According to an embodiment, in operation 1180, the electronic device may store the generated missing biometric data in a memory.

According to various embodiments, an order of the operations of FIG. 11 may be changed and at least some of the operations may be omitted and/or at least one operation (e.g., at least one of the operations of FIGS. 10 and 12 to 14) may be added.

According to an embodiment of the disclosure, it may be identified that the sensor data is missing during the specified cycle or the missing sensor data may be generated and used, using the generative artificial intelligence model.

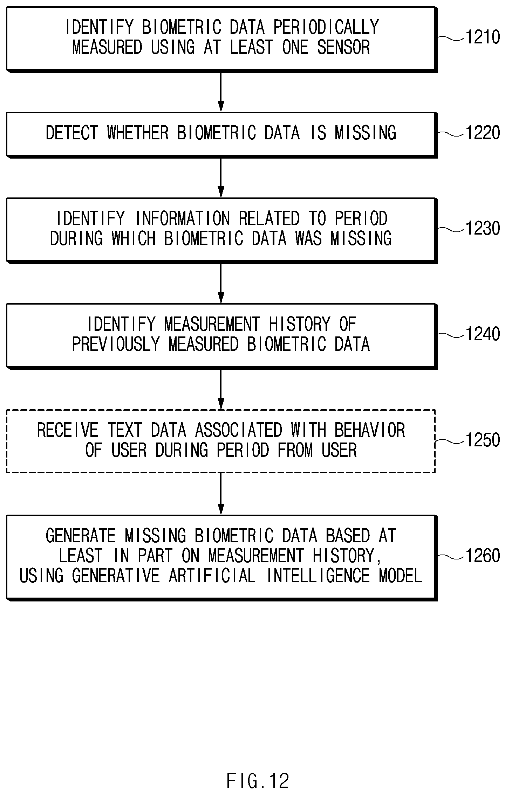

FIG. 12 is a flowchart of a method for managing data of an electronic device according to an embodiment.

In an embodiment below, the respective operations may be sequentially performed, but are not necessarily sequentially performed. For example, an order of the respective operations may be changed and at least two operations may be performed in parallel.

According to an embodiment, operations 1210 to 1260 may be understood as being performed by a processor (e.g., a processor 150 of FIG. 1) of an electronic device (e.g., an electronic device 100 of FIG. 1).

According to an embodiment, in operation 1210, an electronic device (e.g., an electronic device 100 of FIG. 1, an electronic device 210 of FIG. 2, or an electronic device 1501 of FIG. 15) may identify biometric data periodically measured using at least one sensor. For example, a measurement cycle of the biometric data may include, but is not limited to, one day, one month, or one year and may be specified as any period. According to an embodiment, the electronic device may receive biometric data measured using a sensor of an external electronic device (e.g., a wearable device) from the external electronic device.

According to an embodiment, in operation 1220, the electronic device may detect whether the biometric data is missing. For example, when the period is one day, the processor 150 may recognize a day when the biometric data is missing (hereinafter, a "missing day"). According to an embodiment, the processor 150 may determine whether the biometric data is missing based on the result of comparing, for each specified cycle, a time when the biometric data is measured, an operation state of the electronic device (e.g., a time when the electronic device is powered on or a time when the electronic device is powered off, or a time when the sensor is activated/deactivated), or a time when the external electronic device (e.g., the wearable device) is worn with a specified threshold (e.g., threshold time).

According to an embodiment, in operation 1230, the electronic device may identify information related to a period during which the biometric data was missing. For example, the electronic device may identify information associated with the missing day of the biometric data (e.g., a date, weather, a day of the week, and/or a year).

According to an embodiment, in operation 1240, the electronic device may identify a measurement history of previously measured biometric data. For example, the electronic device may identify a measurement history of biometric data measured before the missing day and/or a measurement history of biometric data measured after the missing day. For example, the electronic device may identify a measurement history of biometric data measured during another period corresponding to the period.

According to an embodiment, in operation 1250, the electronic device may receive text data associated with behavior of the user during the period from the user. According to an embodiment, the electronic device may determine whether additional data is required to generate missing biometric data. For example, when the additional data is not required to generate the missing biometric data, the electronic device may omit operation 1250. According to an embodiment, the electronic device may obtain text data from the user through an input through a user interface, an input through a physical key, and/or a voice input through a microphone.

According to an embodiment, in operation 1260, the electronic device may generate missing biometric data based at least in part on the measurement history of the biometric data, using a generative artificial intelligence model (e.g., an AI model 213 of FIG. 2, an AI model 330 of FIG. 3, an AI model 430 of FIG. 4, or an AI model 930 of FIG. 9). According to an embodiment, when receiving the text data in operation 1250, the electronic device may generate the missing biometric data based on the measurement history of the biometric data and/or the text data.

According to various embodiments, an order of the operations of FIG. 12 may be changed and at least some of the operations may be omitted and/or at least one operation (e.g., at least one of operations of FIGS. 10, 11, 13, and 14) may be added.

According to an embodiment of the disclosure, it may be identified that the biometric data is missing during the specified cycle or the missing biometric data may be generated and used, using the generative artificial intelligence model.

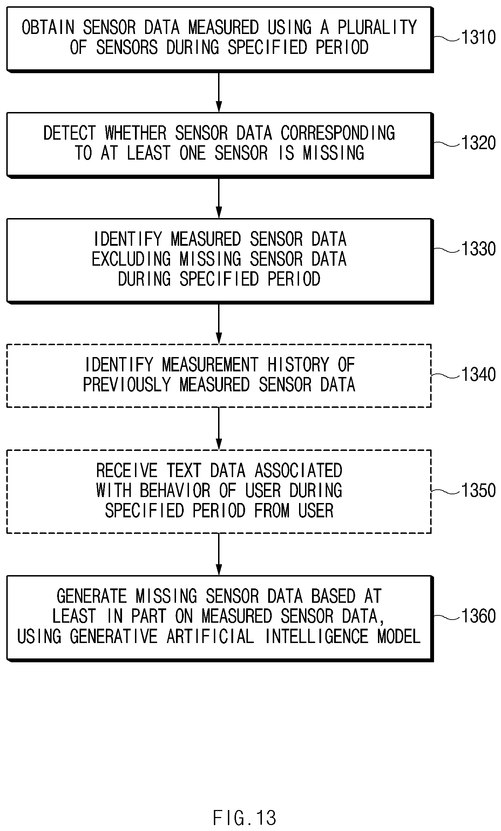

FIG. 13 is a flowchart of a method for managing data of an electronic device according to an embodiment.

In an embodiment below, the respective operations may be sequentially performed, but are not necessarily sequentially performed. For example, an order of the respective operations may be changed and at least two operations may be performed in parallel.

According to an embodiment, operations 1310 to 1380 may be understood as being performed by a processor (e.g., a processor 150 of FIG. 1) of an electronic device (e.g., an electronic device 100 of FIG. 1).

According to an embodiment, in operation 1310, an electronic device (e.g., an electronic device 100 of FIG. 1, an electronic device 210 of FIG. 2, or an electronic device 1501 of FIG. 15) may obtain sensor data measured using a plurality of sensors during a specified period (which may be referred to as the term "session" in the disclosure). For example, the specified period may be specified as a different period based on a user input, a state (e.g., log) of the electronic device an application execution state, a state (e.g., an exercise state, a sleep state, or an activity state) of the user, which is determined by the electronic device.

According to an embodiment, in operation 1320, the electronic device may detect whether sensor data corresponding to at least one sensor is missing. For example, the electronic device may detect whether data of at least one sensor among the plurality of sensors used to generate information (e.g., exercise information or health information) associated with the user in the specified period is missing.

According to an embodiment, in operation 1330, the electronic device may identify measured sensor data excluding missing sensor data during the specified period. For example, the electronic device may identify sensor data measured by other sensors except for a sensor corresponding to the missing sensor data among the plurality of sensors.

According to an embodiment, in operation 1340, the electronic device may identify a measurement history of previously measured sensor data. For example, the electronic device may identify a measurement history of biometric data measured during a previous period corresponding to the specified period (session). According to an embodiment, the electronic device may determine whether additional data is required to generate missing sensor data. For example, when the additional data is not required to generate the missing biometric data, the electronic device may omit operation 1340.

According to an embodiment, in operation 1350, the electronic device may receive text data associated with behavior of the user during the specified period from the user. According to an embodiment, the electronic device may determine whether additional data is required to generate missing sensor data. For example, when the additional data is not required to generate the missing sensor data, the electronic device may omit operation 1350. According to an embodiment, the electronic device may obtain text data from the user through an input through a user interface, an input through a physical key, and/or a voice input through a microphone.

According to an embodiment, in operation 1360, the electronic device may generate missing sensor data based at least in part on the measured sensor data, using a generative artificial intelligence model (e.g., an AI model 213 of FIG. 2, an AI model 330 of FIG. 3, an AI model 430 of FIG. 4, or an AI model 930 of FIG. 9). According to an embodiment, the electronic device may generate the missing biometric data based on sensor data obtained through the other sensor except for the sensor corresponding to the missing sensor data in the same session, sensor data obtained in a different session, and/or the text data obtained from the user.

According to various embodiments, an order of the operations of FIG. 13 may be changed and at least some of the operations may be omitted and/or at least one operation (e.g., at least one of the operations of FIGS. 10 to 12 and 14) may be added.

According to an embodiment of the disclosure, it may be identified that the sensor data is missing during the specified cycle or the missing sensor data may be generated and used, using the generative artificial intelligence model.

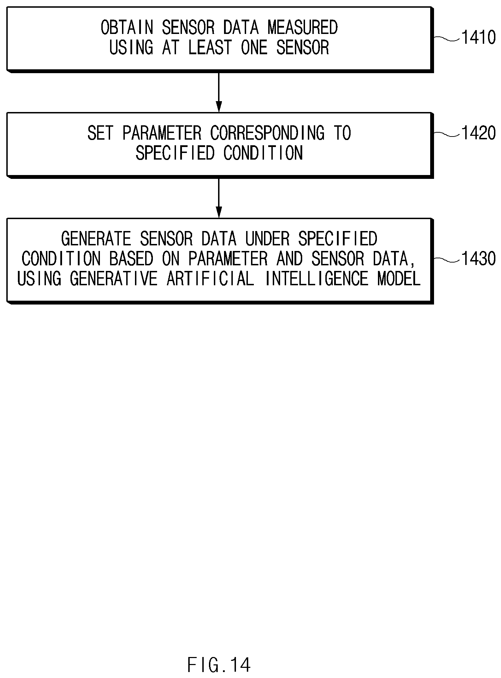

FIG. 14 is a flowchart of a method for managing data of an electronic device according to an embodiment.

In an embodiment below, the respective operations may be sequentially performed, but are not necessarily sequentially performed. For example, an order of the respective operations may be changed and at least two operations may be performed in parallel.

According to an embodiment, operations 1410 to 1430 may be understood as being performed by a processor (e.g., a processor 150 of FIG. 1) of an electronic device (e.g., an electronic device 100 of FIG. 1).

According to an embodiment, in operation 1410, an electronic device (e.g., an electronic device 100 of FIG. 1, an electronic device 210 of FIG. 2, or an electronic device 1501 of FIG. 15) may obtain sensor data measured using at least one sensor. For example, the electronic device may obtain sensor data using a sensor included in the electronic device and/or may receive sensor data from an external electronic device.

According to an embodiment, in operation 1420, the electronic device may set a parameter corresponding to a specified condition. For example, the electronic device may set a parameter corresponding to a virtual health state. For example, as electrocardiogram (ECG) should be measured when irregular heartbeats occur in patients with atrial fibrillation to collect ECG data of atrial fibrillation, it may be possible to obtain desired ECG data. In this case, because the number of the patients with atrial fibrillation is small and a time when irregular heartbeats occur even in the patients with atrial fibrillation is limited, it may be difficult to collect the desired ECG data. The electronic device may set a parameter corresponding to a specified condition (e.g., an atrial fibrillation state) based on information associated with a feature of the atrial fibrillation. For example, the electronic device may set a parameter corresponding to a virtual exercise state (e.g., anaerobic exercise, aerobic exercise, strength exercise, exercise with a lot of movement, or exercise with little movement). For example, sensor data of a sensor (e.g., a PPG sensor) may vary depending on the exercise state.

According to an embodiment, in operation 1430, the electronic device may generate sensor data under the specified condition based on the parameter and the sensor data, using a generative artificial intelligence model (e.g., an AI model 213 of FIG. 2, an AI model 330 of FIG. 3, an AI model 430 of FIG. 4, or an AI model 930 of FIG. 9). For example, the electronic device may apply the set parameter (e.g., the parameter corresponding to the atrial fibrillation state) to the measured sensor data (e.g., ECG data in a normal state) to generate sensor data under the specified condition (e.g., ECG data in the atrial fibrillation state), using the generative artificial intelligence model. For example, the electronic device may generate sensor data corresponding to a virtual exercise state (e.g., anaerobic exercise, aerobic exercise, strength exercise, exercise with a lot of movement, or exercise with little movement), using the generative artificial intelligence model.

According to various embodiments, an order of the operations of FIG. 14 may be changed and at least some of the operations may be omitted and/or at least one operation (e.g., at least one of the operations of FIGS. 10 to 13) may be added.

According to an embodiment, the electronic device may variously generate sensor data corresponding to a virtual condition to variously generate sensor data capable of being used to develop, analyze, and/or evaluate an algorithm even in a limited environment.

A method for managing data of an electronic device according to an embodiment may include identifying biometric data periodically measured using at least one sensor.

According to an embodiment, the method may include determining whether the biometric data is missing.

According to an embodiment, the method may include identifying information related to a period during which the biometric data was missing.

According to an embodiment, the method may include identifying a measurement history of previously measured biometric data.

According to an embodiment, the method may include generating, using a generative artificial intelligence model, missing biometric data based at least in part on the measurement history.

According to an embodiment, the method may include receiving text data associated with behavior of a user during the period from the user.

According to an embodiment, the generating the missing biometric data may include generating the missing biometric data based on the text data and the measurement history.

According to an embodiment, the at least one sensor may include a plurality of sensors.

According to an embodiment, the method may include obtaining sensor data measured using the plurality of sensors during a specified period.

According to an embodiment, the method may include detecting whether sensor data corresponding to at least one sensor among the plurality of sensors is missing.

According to an embodiment, the method may include identifying measured sensor data excluding missing sensor data during the specified period.

According to an embodiment, the method may include generating, using the generative artificial intelligence model, the missing sensor data based at least in part on the measured sensor data.

According to an embodiment, the method may include identifying a measurement history of sensor data measured in a previous measurement period corresponding to the specified period.

According to an embodiment, the generating the missing sensor data may include generating the sensor data based at least in part on the measurement history.

According to an embodiment, the method may include receiving text data associated with behavior of a user during the specified period from the user.

According to an embodiment, the generating the missing sensor data may include generating the sensor data based at least in part on the text data.

According to an embodiment, the method may include obtaining at least some of biometric data or sensor data obtained through a sensor of an external electronic device from the external electronic device.

According to an embodiment, the method may include determining whether data is missing for the obtained at least some of the biometric data or the sensor data.

According to an embodiment, the method may include generating, using the generative artificial intelligence model, missing data for the at least some of the biometric data or the sensor data.

According to an embodiment, the method may include comparing, for each specified cycle, a time when the biometric data is missing with a specified threshold time based on at least one of an amount of a time when the biometric data is measured or an operation state of the electronic device.

According to an embodiment, the method may include determining that the biometric data is missing when the time exceeds the specified threshold time.

According to an embodiment, the method may include outputting, through a display, a user interface including the measured biometric data and information indicating whether the biometric data is missing.

According to an embodiment, the method may include receiving a user input confirming whether to use the generated biometric data through the user interface when the missing biometric data is generated.

According to an embodiment, the method may include storing the generated biometric data in a memory based on the user input for confirming whether to use the generated biometric data.

According to an embodiment, the method may include receiving a user input for modifying the generated missing biometric data or the missing sensor data.

According to an embodiment, the method may include storing the modified missing biometric data or missing sensor data in a memory based on the user input for modifying the biometric data or the sensor data.

According to an embodiment, the method may include obtaining sensor data measured using the at least one sensor.

According to an embodiment, the method may include setting a parameter corresponding to a specified condition.

According to an embodiment, the method may include generating, using the generative artificial intelligence model, sensor data under the condition based on the parameter and the obtained sensor data.

According to embodiments of the disclosure, it may be identified that the biometric data and/or the sensor data are/is missing during the specified cycle or the specified period and the missing biometric data and/or sensor data may be generated and used, using the generative artificial intelligence model. According to embodiments of the disclosure, sensor data corresponding to a virtual condition may be variously generated to variously generate sensor data capable of being used to develop, analyze, and/or evaluate an algorithm even in a limited environment.

FIG. 15 is a block diagram illustrating an electronic device 1501 in a network environment 1500 according to various embodiments. Referring to Fig. 15, the electronic device 1501 in the network environment 1500 may communicate with an electronic device 1502 via a first network 1598 (e.g., a short-range wireless communication network), or at least one of an electronic device 1504 or a server 1508 via a second network 1599 (e.g., a long-range wireless communication network). According to an embodiment, the electronic device 1501 may communicate with the electronic device 1504 via the server 1508. According to an embodiment, the electronic device 1501 may include a processor 1520, memory 1530, an input module 1550, a sound output module 1555, a display module 1560, an audio module 1570, a sensor module 1576, an interface 1577, a connecting terminal 1578, a haptic module 1579, a camera module 1580, a power management module 1588, a battery 1589, a communication module 1590, a subscriber identification module(SIM) 1596, or an antenna module 1597. In some embodiments, at least one of the components (e.g., the connecting terminal 1578) may be omitted from the electronic device 1501, or one or more other components may be added in the electronic device 1501. In some embodiments, some of the components (e.g., the sensor module 1576, the camera module 1580, or the antenna module 1597) may be implemented as a single component (e.g., the display module 1560).

The processor 1520 may execute, for example, software (e.g., a program 1540) to control at least one other component (e.g., a hardware or software component) of the electronic device 1501 coupled with the processor 1520, and may perform various data processing or computation. According to one embodiment, as at least part of the data processing or computation, the processor 1520 may store a command or data received from another component (e.g., the sensor module 1576 or the communication module 1590) in volatile memory 1532, process the command or the data stored in the volatile memory 1532, and store resulting data in non-volatile memory 1534. According to an embodiment, the processor 1520 may include a main processor 1521 (e.g., a central processing unit (CPU) or an application processor (AP)), or an auxiliary processor 1523 (e.g., a graphics processing unit (GPU), a neural processing unit (NPU), an image signal processor (ISP), a sensor hub processor, or a communication processor (CP)) that is operable independently from, or in conjunction with, the main processor 1521. For example, when the electronic device 1501 includes the main processor 1521 and the auxiliary processor 1523, the auxiliary processor 1523 may be adapted to consume less power than the main processor 1521, or to be specific to a specified function. The auxiliary processor 1523 may be implemented as separate from, or as part of the main processor 1521.

The auxiliary processor 1523 may control at least some of functions or states related to at least one component (e.g., the display module 1560, the sensor module 1576, or the communication module 1590) among the components of the electronic device 1501, instead of the main processor 1521 while the main processor 1521 is in an inactive (e.g., sleep) state, or together with the main processor 1521 while the main processor 1521 is in an active state (e.g., executing an application). According to an embodiment, the auxiliary processor 1523 (e.g., an image signal processor or a communication processor) may be implemented as part of another component (e.g., the camera module 1580 or the communication module 1590) functionally related to the auxiliary processor 1523. According to an embodiment, the auxiliary processor 1523 (e.g., the neural processing unit) may include a hardware structure specified for artificial intelligence model processing. An artificial intelligence model may be generated by machine learning. Such learning may be performed, e.g., by the electronic device 1501 where the artificial intelligence is performed or via a separate server (e.g., the server 1508). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 1530 may store various data used by at least one component (e.g., the processor 1520 or the sensor module 1576) of the electronic device 1501. The various data may include, for example, software (e.g., the program 1540) and input data or output data for a command related thereto. The memory 1530 may include the volatile memory 1532 or the non-volatile memory 1534.

The program 1540 may be stored in the memory 1530 as software, and may include, for example, an operating system (OS) 1542, middleware 1544, or an application 1546.

The input module 1550 may receive a command or data to be used by another component (e.g., the processor 1520) of the electronic device 1501, from the outside (e.g., a user) of the electronic device 1501. The input module 1550 may include, for example, a microphone, a mouse, a keyboard, a key (e.g., a button), or a digital pen (e.g., a stylus pen).

The sound output module 1555 may output sound signals to the outside of the electronic device 1501. The sound output module 1555 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 1560 may visually provide information to the outside (e.g., a user) of the electronic device 1501. The display module 1560 may include, for example, a display, a hologram device, or a projector and control circuitry to control a corresponding one of the display, hologram device, and projector. According to an embodiment, the display module 1560 may include a touch sensor adapted to detect a touch, or a pressure sensor adapted to measure the intensity of force incurred by the touch.

The audio module 1570 may convert a sound into an electrical signal and vice versa. According to an embodiment, the audio module 1570 may obtain the sound via the input module 1550, or output the sound via the sound output module 1555 or a headphone of an external electronic device (e.g., an electronic device 1502) directly (e.g., wiredly) or wirelessly coupled with the electronic device 1501.

The sensor module 1576 may detect an operational state (e.g., power or temperature) of the electronic device 1501 or an environmental state (e.g., a state of a user) external to the electronic device 1501, and then generate an electrical signal or data value corresponding to the detected state. According to an embodiment, the sensor module 1576 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1577 may support one or more specified protocols to be used for the electronic device 1501 to be coupled with the external electronic device (e.g., the electronic device 1502) directly (e.g., wiredly) or wirelessly. According to an embodiment, the interface 1577 may include, for example, a high definition multimedia interface (HDMI), a universal serial bus (USB) interface, a secure digital (SD) card interface, or an audio interface.

A connecting terminal 1578 may include a connector via which the electronic device 1501 may be physically connected with the external electronic device (e.g., the electronic device 1502). According to an embodiment, the connecting terminal 1578 may include, for example, a HDMI connector, a USB connector, a SD card connector, or an audio connector (e.g., a headphone connector).

The haptic module 1579 may convert an electrical signal into a mechanical stimulus (e.g., a vibration or a movement) or electrical stimulus which may be recognized by a user via his tactile sensation or kinesthetic sensation. According to an embodiment, the haptic module 1579 may include, for example, a motor, a piezoelectric element, or an electric stimulator.

The camera module 1580 may capture a still image or moving images. According to an embodiment, the camera module 1580 may include one or more lenses, image sensors, image signal processors, or flashes.

The power management module 1588 may manage power supplied to the electronic device 1501. According to one embodiment, the power management module 1588 may be implemented as at least part of, for example, a power management integrated circuit (PMIC).

The battery 1589 may supply power to at least one component of the electronic device 1501. According to an embodiment, the battery 1589 may include, for example, a primary cell which is not rechargeable, a secondary cell which is rechargeable, or a fuel cell.

The communication module 1590 may support establishing a direct (e.g., wired) communication channel or a wireless communication channel between the electronic device 1501 and the external electronic device (e.g., the electronic device 1502, the electronic device 1504, or the server 1508) and performing communication via the established communication channel. The communication module 1590 may include one or more communication processors that are operable independently from the processor 1520 (e.g., the application processor (AP)) and supports a direct (e.g., wired) communication or a wireless communication. According to an embodiment, the communication module 1590 may include a wireless communication module 1592 (e.g., a cellular communication module, a short-range wireless communication module, or a global navigation satellite system (GNSS) communication module) or a wired communication module 1594 (e.g., a local area network (LAN) communication module or a power line communication (PLC) module). A corresponding one of these communication modules may communicate with the external electronic device via the first network 1598 (e.g., a short-range communication network, such as Bluetooth^{™}, wireless-fidelity (Wi-Fi) direct, or infrared data association (IrDA)) or the second network 1599 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1592 may identify and authenticate the electronic device 1501 in a communication network, such as the first network 1598 or the second network 1599, using subscriber information (e.g., international mobile subscriber identity (IMSI)) stored in the subscriber identification module 1596.

The wireless communication module 1592 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 1592 may support a high-frequency band (e.g., the mmWave band) to achieve, e.g., a high data transmission rate. The wireless communication module 1592 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 1592 may support various requirements specified in the electronic device 1501, an external electronic device (e.g., the electronic device 1504), or a network system (e.g., the second network 1599). According to an embodiment, the wireless communication module 1592 may support a peak data rate (e.g., 20Gbps or more) for implementing eMBB, loss coverage (e.g., 1564dB or less) for implementing mMTC, or U-plane latency (e.g., 0.5ms or less for each of downlink (DL) and uplink (UL), or a round trip of 15ms or less) for implementing URLLC.

The antenna module 1597 may transmit or receive a signal or power to or from the outside (e.g., the external electronic device) of the electronic device 1501. According to an embodiment, the antenna module 1597 may include an antenna including a radiating element composed of a conductive material or a conductive pattern formed in or on a substrate (e.g., a printed circuit board (PCB)). According to an embodiment, the antenna module 1597 may include a plurality of antennas (e.g., array antennas). In such a case, at least one antenna appropriate for a communication scheme used in the communication network, such as the first network 1598 or the second network 1599, may be selected, for example, by the communication module 1590 (e.g., the wireless communication module 1592) from the plurality of antennas. The signal or the power may then be transmitted or received between the communication module 1590 and the external electronic device via the selected at least one antenna. According to an embodiment, another component (e.g., a radio frequency integrated circuit (RFIC)) other than the radiating element may be additionally formed as part of the antenna module 1597.

According to various embodiments, the antenna module 1597 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 1501 and the external electronic device 1504 via the server 1508 coupled with the second network 1599. Each of the electronic devices 1502 or 1504 may be a device of a same type as, or a different type, from the electronic device 1501. According to an embodiment, all or some of operations to be executed at the electronic device 1501 may be executed at one or more of the external electronic devices 1502, 1504, or 1508. For example, if the electronic device 1501 should perform a function or a service automatically, or in response to a request from a user or another device, the electronic device 1501, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1501. The electronic device 1501 may provide the outcome, with or without further processing of the outcome, as at least part of a reply to the request. To that end, a cloud computing, distributed computing, mobile edge computing (MEC), or client-server computing technology may be used, for example. The electronic device 1501 may provide ultra low-latency services using, e.g., distributed computing or mobile edge computing. In another embodiment, the external electronic device 1504 may include an internet-of-things (IoT) device. The server 1508 may be an intelligent server using machine learning and/or a neural network. According to an embodiment, the external electronic device 1504 or the server 1508 may be included in the second network 1599. The electronic device 1501 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1540) including one or more instructions that are stored in a storage medium (e.g., internal memory 1536 or external memory 1538) that is readable by a machine (e.g., the electronic device 1501). For example, a processor (e.g., the processor 1520) of the machine (e.g., the electronic device 1501) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device, comprising:
communication circuitry;
at least one sensor;
memory configured to store a generative artificial intelligence model; and
a processor,
wherein the memory stores instructions that, when executed by the processor, cause the electronic device to:
identify biometric data periodically measured using the at least one sensor;
determine whether the biometric data is missing;
identify information related to a period during which the biometric data was missing;
identify a measurement history of previously measured biometric data; and
using the generative artificial intelligence model, generate missing biometric data based at least in part on the measurement history.

2. The electronic device of claim 1, wherein the instructions, when executed by the processor, cause the electronic device to:
receive text data associated with behavior of a user during the period; and
generate the missing biometric data based on the text data and the measurement history.

3. The electronic device of claim 1, wherein the at least one sensor comprises a plurality of sensors, and
wherein the instructions, when executed by the processor, cause the electronic device to:
obtain sensor data measured using the plurality of sensors during a specified period;
determine whether sensor data corresponding to at least one sensor among the plurality of sensors is missing;
identify measured sensor data excluding missing sensor data during the specified period; and
using the generative artificial intelligence model, generate missing sensor data based at least in part on the measured sensor data.

4. The electronic device of claim 3, wherein the instructions, when executed by the processor, cause the electronic device to:
identify a measurement history of sensor data measured in a previous measurement period corresponding to the specified period; and
generate the missing sensor data based at least in part on the measurement history of the sensor data.

5. The electronic device of claim 3 or 4, wherein the instructions, when executed by the processor, cause the electronic device to:
receive text data associated with behavior of a user during the specified period; and
generate the missing sensor data based at least in part on the text data.

6. The electronic device of claim 1 or 3, wherein the instructions, when executed by the processor, cause the electronic device to:
obtain at least some of biometric data or sensor data obtained through at least one sensor of an external electronic device from the external electronic device;
determine whether data is missing for the at least some of the biometric data or the sensor data; and
using the generative artificial intelligence model, generate missing data for the at least some of the biometric data or the sensor data.

7. The electronic device of claim 1, wherein the instructions, when executed by the processor, cause the electronic device to:
compare, for each specified cycle, a time when the biometric data is missing with a specified threshold time based on at least one of an amount of a time when the biometric data is measured or an operation state of the electronic device; and
when the time exceeds the specified threshold time, determine that the biometric data is missing.

8. The electronic device of claim 1, wherein the instructions, when executed by the processor, cause the electronic device to:
output, through the display, a user interface including the measured biometric data and information indicating whether the biometric data is missing;
when the missing biometric data is generated, receive a user input confirming whether to use the generated biometric data through the user interface; and
store the generated biometric data in the memory based on the user input.

9. The electronic device of claim 1 or 3, wherein the instructions, when executed by the processor, cause the electronic device to:
receive a user input for modifying the generated missing biometric data or the missing sensor data; and
store the modified missing biometric data or missing sensor data in the memory based on the user input.

10. The electronic device of claim 1, wherein the instructions, when executed by the processor, cause the electronic device to:
obtain sensor data measured using the at least one sensor;
set a parameter corresponding to a specified condition; and
using the generative artificial intelligence model, generate sensor data under the specified condition based on the parameter and the obtained sensor data.

11. A method for managing data of an electronic device, the method comprising:
identifying biometric data periodically measured using at least one sensor;
determining whether the biometric data is missing;
identifying information related to a period during which the biometric data was missing;
identifying a measurement history of previously measured biometric data; and
generating, using a generative artificial intelligence model, missing biometric data based at least in part on the measurement history.

12. The method of claim 11, further comprising:
receiving text data associated with behavior of a user during the period,
wherein the generating the missing biometric data comprises generating the missing biometric data based on the text data and the measurement history.

13. The method of claim 11, wherein the at least one sensor comprises a plurality of sensors, and
further comprising:
obtaining sensor data measured using the plurality of sensors during a specified period;
determining whether sensor data corresponding to at least one sensor among the plurality of sensors is missing;
identifying measured sensor data excluding missing sensor data during the specified period; and
generating, using the generative artificial intelligence model, the missing sensor data based at least in part on the measured sensor data.

14. The method of claim 13, further comprising:
identifying a measurement history of sensor data measured in a previous measurement period corresponding to the specified period,
wherein the generating the missing sensor data comprises generating the missing sensor data based at least in part on the measurement history of the sensor data.

15. The method of claim 13 or 14, further comprising:
receiving text data associated with behavior of a user during the specified period,
wherein the generating the missing sensor data comprises generating the missing sensor data based at least in part on the text data.
